(19) Europäisches Patentamt
European Patent Office
Office européen des brevets

(11) EP 4 592 676 A1

(12) EUROPEAN PATENT APPLICATION

(43) Date of publication:
30.07.2025 Bulletin 2025/31

(21) Application number: 24154434.5

(22) Date of filing: 29.01.2024

(51) International Patent Classification (IPC):
*G01N 33/50* (2006.01) *G01N 33/557* (2006.01)
*G01N 33/68* (2006.01) *B01L 3/00* (2006.01)

(52) Cooperative Patent Classification (CPC):
G01N 33/557; G01N 33/5008; G01N 33/68

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(71) Applicant: Saber Bio SAS
75013 Paris (FR)

(72) Inventor: Gérard, Annabelle
91120 Paris (FR)

(74) Representative: CH Kilger Anwaltspartnerschaft mbB
Fasanenstraße 29
10719 Berlin (DE)

# (54) METHODS AND SYSTEMS FOR SCREENING LIGANDS AND RECEPTORS

(57) The present invention provides methods for identifying a compound having a specific biological function, such as its binding affinity to a target or capability of being internalized within a cell. The selection and combination of specific parameters, which comprise the limit of detection of the assay and the concentration of the target, allows carrying out the methods.

EP 4 592 676 A1

## Description

### Field of the invention

The present invention is in the field of cellular and molecular biology and relates to methods for identifying a compound having a specific biological function. The present invention is also in the field of microfluidics and relates to microfluidic systems and their use for carrying out biological assays.

### Background of the invention

Molecules show an innate affinity for one another due to electrostatic forces, hydrogen bonds and dispersion forces. The non-covalent interactions that result from this affinity are of particular importance in biological processes, including the catalysis of chemical reactions by enzymes, neutralization of pathogens by antibodies and/or T cells and stimulation of cellular activities by hormones, cytokines, chemokines and in general any interaction between two molecules. The interactions between a biological receptor with its natural ligand can be defined in terms of different properties such as specificity, affinity, saturation, binding constants and physiological response.

In the context of therapeutic antibodies, affinity is one of the key properties characterizing the potency and to some extend the mechanism of action, the pharmaco-dynamics and pharmaco-kinetics of this class of macromolecules. Antibody affinity describes the intensity with which a single antibody binds to its specific epitope in an antigen. Therefore, antibodies with higher affinities may allow lower doses or longer intervals of administration during therapy. Also, as antibodies require sophisticated production systems and therapeutic doses, a high affinity may have a significant impact on the commercial success of a therapeutic antibody drug. In some cases, a lower affinity may be desired. For example, it has been recently developed a new approach for vaccines against coronaviruses using modified spike proteins with lower binding affinity (Ratswohl et al. Eur. J. Immunol. 2023, 0: 2350408). In addition, in contrast to naturally occurring or direct-targeting therapeutic antibodies, immunomodulatory antibodies, which are designed to modulate receptor signalling, low rather than high affinity delivers greater activity through increased clustering. Such approach delivered higher immune cell activation, in vivo T cell expansion and antitumour activity (Yu et al., Nature, 2023, 614:539-547).

A further property characterizing therapeutic antibodies lies in their ability to internalize into cells, rather than just binding to antigens on the cell surface (Dumontet et al., Nat Rev Drug Discov 2023, 22(8):641-661). Antigen-mediated antibody internalization plays an important role in several antibody-based therapeutics as it allows delivery of drugs to cancer cells via antibody drug conjugates (ADC), removal or degradation of surface receptors from cancer cells and antibody-based immunotherapies to identify tumour cells for immune cell killing (*e.g.*, antibody-dependent cellular cytotoxicity (ADCC), antibody-dependent cellular phagocytosis (ADCP)).

Methods for assessing antibody affinity known in the art are based on kinetic analysis alone (Guo et al. RSC Adv. 2016, 6:13837-13845) or in combination with other techniques (Lupu et al. Int. J. Mol. Sci. 2021, 22(23):12832). Droplet-based microfluidic assays also allow characterization of antigen specific antibody-secreting cells with high throughput. Gérard *et al.* describe a droplet-based microfluidics platform for antibody screening at the single cell level using a double fluorescent sandwich ELISA for the screening, identification, sorting and $V_H$-$V_L$ paired sequencing of antigen-specific IgG antibodies produced by antibody-secreting cells from immunized mice (Gérard et al. Nat. Biotechnol. 2020, 38(6):715-721). Other approaches to study antibody-secreting cells in droplets consist of collecting droplets in a horizontal plane and measure changes in droplet fluorescence over multiple timepoints by stationary droplet-based assay (Bucheli et al. Eur. J. Immunol. 2021, 51(6):1334-1347) or DropMap (Eyer et al. Nat. Biotechnol. 2017, 35(10):977-982; Canales-Herrerias et al. J. Clin. Invest. 2022, 132(12):e153580). Beacon® (Berkeley Lights, now part of Bruker) and Cyto-Mine® (Sphere Fluidics) commercial platforms represent further approaches for investigating therapeutic antibodies.

However, current methods for characterizing antibodies affinities and internalization are post-screening process that require prior identification of the antibodies before being tested to determine their affinity (*e.g.*, by Octet® SPR analysis) or internalization property (*e.g.*, by flow cytometry analysis).

Also, discovery of therapeutically and/or functionally relevant antibodies requires screening of antibody secreting cells (primary or engineered ones). Nevertheless, the frequency of cells secreting relevant antibodies is relatively low (~1% or below), mainly due to the large diversity of antibody repertoire and low immune response.

Additionally, antibodies need to be diluted to working concentrations for different applications. The final working concentration is critical to the precision and accuracy of each assay. The optimal final concentration is a balance among the assay sensitivity, the affinity of the antibody for its epitope and the non-specific binding between the antibody and other antigens (*i.e.*, the cross-reactivity) that generates the background noise. If the working concentration is too low, the target protein or antigen might not be detectable. If the working concentration is too high, the background noise might be high resulting in a poor signal-to-noise ratio. Therefore, the proper dilution for a working concentration needs to be optimized for each antibody, application and sample to obtain the best signal-to-noise ratio. While some antibodies have a wide range of acceptable working concentrations, many antibodies produce an acceptable signal-to-noise ratio only at a precise

concentration. Optimization of assay conditions such as protein/reagent concentration, and/or antibody dilution are laborious and time-consuming.

The method according to the present invention is intended to solve the drawbacks and limitations affecting the current assays by providing with a direct method for screening compound-target interactions. In particular, the method disclosed herein allows identification of a compound in terms of its specific biological function by selecting assay limit of detection (LOD) and target concentration, without requiring adjustment or calculation of the optimal working concentration (*e.g.*, dilution) of the compound.

**Summary of the invention**

In one aspect, the present invention provides with a method for identifying and classifying a compound being able to bind a target, in an assay having a limit of detection (LOD) for said compound, the method comprising the steps of:

(a) providing a microreactor comprising a compound and a target,
(b) incubating said compound and said target under conditions sufficient to allow the formation of a complex between said compound and said target,
(c) identifying said complex by means of detecting a signal above or below a first threshold or a second threshold, wherein:

(i) if the signal is below the first threshold, and said target is present in a concentration equal to or up to five-fold greater than said LOD, said compound is classified as having the highest binding affinity for said target;
(ii) if the signal is above the first threshold, and said target is present in a concentration at least five-fold greater than said LOD, said compound is classified as having the lowest binding affinity for said target;
(iii) if the signal is above the second threshold, and said target is present in a concentration lower than said LOD, said compound is classified as exhibiting an internalization capacity,

wherein the limit of detection (LOD) of the compound to be identified and classified ranges from about 0.01 nM to about 10 nM.

In another aspect, the present invention provides with a system for screening a compound in an assay having a predetermined limit of detection (LOD), the system comprising:

(a) a first module configured for collecting a compound and a target;
(b) a second module configured for incubating said compound and said target;
(c) a third module configured for detecting an interaction between said compound and said target;
(d) a fourth module configured for screening said compound.

**Brief description of the figures**

Figure 1 shows detectable compound affinities (x-axis), as a function of initial compound concentration (y axis, $IgG_0$) and target concentration (y axis, Ag) in systems having a LOD of 1 nM (A and B) and 0.2 nM (C and D). Selecting an assay with a LOD of 1 nM: using 5 nM of antigen ($Ag_0$) for assay readout and cells secreting 2 nM or 5 nM of antibody ($IgG_0$), which correspond to median and mean primary B cell secretion rate (in 80pL droplet after 1h secretion), respectively, the antibody affinity detected is below 4 or 12 nM. Working at higher concentration of antigen ($Ag_0$ = 30 nM) the method enables screening of antibodies with affinity from 30 nM up to 95 nM (see Figure 1A-B). With reference to an assay with a LOD of 0.2 nM the method enables detecting antibodies with a broad affinity range (high to low affinity) (see Figure 1C-D).

Figure 2 shows detectable compound affinities, as a function of initial compound concentration (x axis) and target concentration (y axis) in a system having a LOD of 1 nM. The figure shows examples with variable antibody concentration ($IgG_0$, nM) and antigen (Ag) concentration ($Ag_0$, nM). The curved lines delimit the maximum detectable KD (up to 10 nM, 50 nM, 100 nM, 500 nM and above) in different conditions of $IgG_0$ and $Ag_0$, in a system having a LOD of 1 nM.

Figure 3 shows detectable compound affinities, as a function of initial compound concentration (x axis) and target concentration (y axis) in a system having a LOD of 1 nM. Selecting an assay with a LOD of 1 nM and using 5 nM antigen ($Ag_0$) for assay readout, and cells secreting 2 nM or 5 nM of antibody ($IgG_0$), which correspond to about median and

mean primary plasma B cells secretion rate (in 80pL droplet after 1h secretion), respectively, the method will enrich positive event for antibodies having an affinity below 16 nM.

Figure 4 shows detectable compound affinities, as a function of initial compound concentration (x axis) and target concentration (y axis) in a system having a LOD of 1 nM. Selecting an assay with a LOD of 1 nM and using 10 nM antigen ($Ag_0$) for assay readout, and cells secreting 2 nM or 5 nM of antibody ($IgG_0$), which correspond to about median and mean primary plasma B cells secretion rate (in 80pL droplet after 1h secretion), respectively, the method will report positive event for antibodies having an affinity below 50 nM.

Figure 5 shows detectable compound affinities, as a function of initial compound concentration (x axis) and target concentration (y axis) in a system having a LOD of 1 nM. Selecting an assay with a LOD of 1 nM and using 15 nM antigen ($Ag_0$) for assay readout, and cells secreting 2 nM or 5 nM of antibody ($IgG_0$), which correspond to about median and mean primary plasma B cells secretion rate (in 80pL droplet after 1h secretion), respectively, the method will report positive event for antibodies having mid to low of affinities.

Figure 6 shows the screening of antibodies by the method according to the present invention in terms of their ability to internalize into cells. The figure shows assay where compound of interest binds to a cell/particle. In 'standard' assay condition (left panel), binding of compound to cell/particle vs. internalization, internalized compound led to increase of peak height fluorescent signal (bottom panel). In an 'internalization assay' specific condition (right panel), only the internalization of the compound inside the cell will lead to detectable fluorescent signal (bottom panel). These examples are assuming: a cell diameter of 10 $\mu$m; surface of the cell = 166$\pi$; internalization from receptor mediated endocytic pathways, with ultimately degradation in lysosome. Endosomes have a diameter of 50 nm and lysosomes of 1 $\mu$m. Internalization of receptor-Ab would relocalize to an organelle of ~ 3 $\mu$m (at max). Surface of organelle/internalization = 4.5$\pi$. The signal intensity is thus expected to be ~ 37 times higher/more concentrated when receptor/antibody are internalized. It is thus possible to design an assay for internalization specific antibodies, with detection molecule concentration just below LOD concentration.

Figure 7 shows the screening of antibodies by the method according to the present invention in terms of their ability to internalize into cells. The figure shows assay where compound of interest binds to a cell/particle and different representation of results, incl. data reporting and analysis to identify internalized specific compound. In 'standard' assay condition (left panel), binding of compound to cell/particle vs. internalization, internalized compound led to increase of maximum fluorescent signal (Max Target Fluo, bottom panel), while decreasing 'area under the curve' (Area Target Fluo). It is thus difficult to identify specifically internalized compound. In an 'internalization assay' specific condition (right panel), the internalization of the compound inside the cell will lead to detectable fluorescent signal (Max Target Fluo and Area Target Fluo, bottom panel). Detecting internalized specific compound is facilitated in this internalizing specific assay.

Figure 8 shows the internalization assay format. Figure 8A represents different assays format, where container (1), target expressing cell/particle (2), solution present in the container (3) and optionally cell/particle producing compound having biological function (4) are incubated. Figure 8B - Examples of compound localization/detection. Figure 8B represents LOD determining assays format and behavior of internalized target/compound of interest/detection agent. In this example, target expressing cell/particle (2), compound of interest (8), detection agent bound to compound of interest (9) and target is bound (5) to cell membrane are incubated. In the left panel, compound of interest is bound to target cell expressed at the surface, together with the detection agent. In the middle panel, compound of interest is internalized inside the target cell, together with the detection agent. In the right panel, compound of interest has either been degraded/recycled to cell surface and only detection agent is still inside the cell/detectable due to internalization process. Figure 8C - Example of different internalization localization. Following internalization of compound, likely together via crossing the cell/particle membrane (6) expressing target bound to cell surface (5), inside cell/particle, the compound of interest/the detection agent bound to it can relocalize to different intracellular compartment (7) having different shapes as depicted.

Figure 9 shows an assay comprising a beadline for capturing a compound of interest binding to its target. Figure 9A - Example of screening format for compound of interest using a beadline assay. In certain condition ligand and receptor are in solution. Detecting interaction requires an assay format compatible with such molecules. The use of paramagnetic bead particle (i.e., beadline assay) can detect interaction and affinity of compound to its ligand. In a container (1), a cell having a biological function (4) present in solution (3) and producing a compound of interest (8) are incubated with a capture agent (10) interacting directly or indirectly with compound of interest (8), which has an affinity for binding its target (11). Interaction of the target (11) to compound of interest (8) is detected by using directly or indirectly labelled

target (11) and directly or indirectly via labelled directly or indirectly detection agent (9). Figure 9B - Distinguishing high affinity vs low affinity compound against target. Represent an example of screening in a container (1) containing a fluid (3) and a compound of interest (8) having binding affinity against a soluble target (11) and produced by a cell having a biological function (4). The assay using beadline format consists of a plurality of capture agents (10). Secretion of compound of interest (8) by a cell having a biological function (4) and its binding to the capture agents (10) is detected by the detection agent bound to compound of interest (9). Depending on the secretion rate of cell having a biological function (4) (and/or amount of compound of interest (8)), some detection agent (9) in solution can be detectable. In condition where LOD of system is low (example 1 nM) and target concentration is low, in condition (a), the compound of interest has high affinity against the target, it tightly binds to the target (high $K_{on}$ and low $K_{off}$), thus 'capturing' all target in solution (11') onto the capture agents (10) bound compound of interest (8). Binding event will be detected in such condition. In condition where LOD of system is low (example 1 nM) and target concentration is low, the compound of interest has low affinity against the target, it does not bind tightly to the target (low $K_{on}$ and high $K_{off}$), thus target in solution (11') is in excess compared to its form bound (11) to compound of interest (8). Binding event will not be detected in such condition (see, Figure 9B).

Figure 10 shows a setup of system and assay LOD. The figure shows examples for determining assay LOD. In (a) a beadline assay LOD is performed, in (b) a single particle assay LOD mimicking a cell is performed and in (c) a single particle assay LOD mimicking a smaller cell is performed. Assay LOD is performed using the similar assay material: (optionally paramagnetic) particles (14) concentration and preparation methods, or particle (14) having size mimicking a cell as well as solution (3) in the container (1). To minimize biological interference, the particles are functionalized with fluorescent molecules (ideally with the same dye as used in the functional assay). The functionalization can be performed by having a detecting particle (13) directly bound to the particle (14) and/or indirectly bound via a capture agent (10) and a reporting molecule (12). Examples of capture agent and/or reporting molecules includes antibodies and derivatives, proteins, lipid, carbohydrates, nucleic acids, chemicals.

Figure 11 shows how to determine the LOD of the assay using beadline format. Assay is performed according to Example 1. Histogram plot for PMT3 signal for each droplet population for the 200 Hz (A), 1 KHz (B) and 2 KHz (C) frequencies are indicated. Calculated average LOD ~ 1.6 nM (D) calculated from linear regression of signal intensities (mean blank + 3 sigma).

Figure 12 shows example of system for compartmentalizing target and reagent mix, performing an assay (reactor), detecting, analyzing, and enriching a target specific compound. The compartmentalizing module is combining reagents and compound in the same compartment, where assays can be performed. The reactor module is taking input from the compartmentalizing module to initiate the assay reaction, for instance by heating up/cooling down the compartments. The detector module is scanning/reading assays readout (using for example fluorescent molecules excitation means and detector means). The assay analyzer is computing the information received from the detectors to visualize and interpret the compound function based on signal emitted by readout molecules and detector means. Both detector and analyzer can modulate on system LOD - increase it by increasing excitation power and detection sensitivity/gain and/or decrease by lowering excitation power and detection sensitivity/gain. Signal processing and computation methods can impact system LOD, for instance by removing electronic noise, by compensating for spill over/stray light. Analyzer, will detect background signal and assay specific signal, analyzer and/or user can define assay threshold to define assay specific signal. Based on the signal detected by detectors (and optionally displayed on a user interface), and when such assay signal is above defined assay threshold, the enrichment module will be able to enrich the material having specific function, as defined having a signal above said threshold. Standard assay selectivity are reaction volume and reaction time, here key inflexion points are dictated by assay LOD and system LOD to be able to specifically enrich/detect target specific compound having specific function. Assay LOD depends on the reporter molecule/assay readout (both in terms of the report identity and number), the system LOD depends on optical setup (both excitation and detection path definition), as well as signal processing.

Figure 13 shows typical results of cell-based assay LOD determination using different readout system (both detector and analyzer). (A) Gating strategy during droplet scanning for detection of droplet containing viable cell having specific signal at cell surface. From left to right: droplet as selected based on size (y axis) over time (x axis), then droplet demultiplexing is performed based on PMT5 channel detection of the different Dy754 dropcode concentration, while having the same width (x axis), among those droplet the one containing alive (not having incorporated NucGreen, thus PMT2 negative) cells (being Cell Trace Violet labelled, thus PMT1 positive) are selected for further analysis of antibody binding to cell (*i.e.,* a PMT4 signal above background). (B) Following similar gating strategy as (A), examples of antibody binding to cell (below histograms) are shown for different primary antibody concentration (from 0 to 10 nM). Upper graphs are showing histogram plot of antibody signal (PMT4) in the droplet non containing cells (*i.e.*, PMT1

negatives). In those conditions, LOD is 1.09*10e6 fluorescence unit reported from PMT for such cell-based assay (*i.e.*, mean blank + 3 standard deviation). Using same amount of reporting molecule, this corresponds to an assay LOD capable of detecting as low as ~ 0.7 nM primary antibody (as estimated by plotting a linear regression of the 1 nM and 10 nM values). (C) Brightfield, Dy754 (indicative of dropcode/droplet conditions), AF647 (indicative of antibody signal), CTV (indicative of presence of cells), and NucGreen (indicative of cell viability) images of droplet for assessing cell-based assay LOD using reporter cells. (D) Quantification of LOD assessment in static images. From left to right: droplet conditions identification using DY754 (increasing Dy754 reflects increasing primary antibody concentration, from 0 to 10 nM as indicated), assessment of droplet diameter uniformity, identification of droplet containing cells (CTV positives), identification of droplet containing live cells (NucGreen negatives) and finally quantification of cell membrane (CM) staining of primary antibody binding using AF647 labelled detection molecule. In those conditions, LOD is 7,151 fluorescence unit reported from images acquisition for such cell-based assay (i.e., mean blank + 3 standard deviation). Using same amount of reporting molecule, this corresponds to an assay LOD capable of detecting as low as ~ 1.0 nM primary antibody (as estimated by ploting a linear regression of the 1 nM and 10 nM values).

Figure 14 shows enrichment of compound having specific function based on LOD and target concentration. (A) Graphs show on the left distribution of IgG secretion (in nM) frequency as per data published in Eyer et al., Nature Biotechnology 2017, 35(10):977-982 and Molari et al., eLife 2020, 9:e55678; as well as cell count (estimating $10^6$ cells being screened) distribution of antibody affinities. (B) Distribution of recovered functionally specific antibodies, considering cell secretion and antibody affinity (both being independent from each other, as per Eyer et al., Nature Biotechnology 2017, 35(10):977-982) shown in A, in an assay having an LOD of 1 nM and with 20 nM of target. Most of the antibodies (> 99,999 %) have medium to low affinity. In conditions where antibody concentration is constant/selected using cell secretion threshold (*i.e.*, about 5 nM, darker histogram plots), the critical KD (KDc) is 76 nM (*i.e.*, maximal Kd that can be detected in such condition). In those conditions, the assay retains a large majority of medium/low affinity antibodies. (C) Distribution of recovered functionally specific antibodies, considering cell secretion and antibody affinity (both being independent from each other, as per Eyer et al., Nature Biotechnology 2017, 35(10):977-982) shown in A, in an assay having an LOD of 1 nM and with 5 nM of target. In those conditions, maximum Kd detected is 100 nM, with significant enrichment of high affinity antibody. In conditions where antibody concentration is constant/selected using cell secretion threshold (*i.e.*, about 5 nM, darker histogram plots), the critical KD is 16 nM. In those conditions, the assay retains a large majority of high affinity antibodies.

Figure 15 is showing statistical data from histogram plot of figure 14. According to set critical $K_D$ (KDc), according to LOD and target concentration ($Ag_0$), efficiency (*i.e.*, % of cells recovered having KD < KDc based on total pool of cells having KD < KDc) and purity (*i.e.*, % cells having specific function over total recovered cells) are impacted. It is thus possible to design an assay to highly enrich for specific function based on assay LOD and antigen concentration, independently of compound concentration.

Figure 16 shows computation of antibody affinity and concentration distribution according to set critical KD (KDc). KDc is a user defined KD for which screening of compound having an affinity below KDc (or above) would be performed. Because KDc depends on target concentration ($Ag_0$), assay LOD and compound concentration ($IgG_0$), it is possible to screen molecule of interest by varying $Ag_0$, while having $IgG_0$ similar. $IgG_0$ can be estimated using a reporting molecule. At mean/median concentration of compound ($IgG_0$), reporting molecule signal will cover most of the signal population (*i.e.*, concentration of compound follows a gaussian distribution - see figure 14A). Horizontal line represents KDc threshold, vertical dashed line (2 nM) is the median compound concentration obtained in assay condition, vertical plain line (5 nM) is the median compound concentration obtained in assay condition. (A) KDc is set at 16 nM by performing an assay having LOD of 1 nM and by using $Ag_0$ at 5 nM, while screening the condition where compound molecules are secreted at mean or median population. The target population of compound of interest is as low as 0.2% (dark grey). (B) KDc is set at 36 nM by performing an assay having LOD of 1 nM and by using $Ag_0$ at 10 nM, while screening the condition where compound molecules are secreted at mean or median population. The target population of compound of interest is as low as 1.2 % (dark grey). (C) KDc is set at 76 nM by performing an assay having LOD of 1 nM and by using $Ag_0$ at 15 nM, while screening the condition where compound molecules are secreted at mean or median population. The target population of compound of interest is as low as 2.5 % (dark grey).

## Detailed description of the invention

The interactions between two binding molecules, such as a receptor with its ligand, are characterized by two important parameters: affinity and concentration. In the context of antibodies, antibody affinity can be described as the strength of the reversible association between an antibody (Ab) and an antigen (Ag) by the following formula:

$$[Ab] + [Ag] \underset{K_{off}}{\overset{K_{on}}{\rightleftarrows}} [Ab \cdot Ag]$$

The strength of antibody-antigen binding is enhanced by a fast association rate, which is proportional to the association rate constant ($k_{on}$ or $k_a$), and by a slow dissociation rate, which is proportional to the dissociation rate constant ($k_{off}$ or $k_d$). The value of affinity is most frequently described by the equilibrium dissociation constant ($K_D$).

Antibody affinities are relatively difficult to be determined using current methods, such as surface plasmon resonance (SPR) or biolayer interferometry (BLI), as these may still include detection of some non-related antibodies. Also, current methods present several limitations. In terms of costs, many of these techniques require specialized equipment and consumables, making them relatively expensive to set up and maintain. In this context, some affinity measurement methods may require a relatively large amount of purified antibody and/or antigen for the experiments. This can be a limitation when working with limited or precious samples, especially if the antibody or antigen is difficult to produce or purify. In terms of time-consuming, some affinity measurement techniques, such as SPR or BLI, involve multiple steps and require several hours to complete a single experiment. This extended timeframe may not be suitable for situations requiring rapid screening or evaluation of large antibody panels. Moreover, some methods, such as SPR and BLI, may face challenges when working with complex biological samples, such as serum or crude cell lysates, due to potential interference from other components present in the sample. Special considerations or additional steps may be necessary to address these challenges.

The method disclosed herein provides with a direct and reliable approach for the identification of a compound of interest having a specific biological function that does not require any pretreatment, such as adjustment or calculation of the optimal working concentration (*e.g.*, dilution) . This finding offers several advantages as the method allows to discriminate compounds based on their affinity in solution using complex biological samples, such as using cellular composition. The method finds applications in screening immune cells producing antibodies in patient suffering from cancer and/or in immunized animal, monitoring the antibody response in patients over time following the administration of therapeutic antibodies, determining the presence of autoantibodies in autoimmune disease or assessing the potency of a monoclonal antibody following its generation, or assessing the clonality of antibody producing cells.

In particular, the inventors have found that the selection of an assay with a predetermined limit of detection (LOD) along with the selection of the target concentration allows to screen a compound of interest capable of binding said target by its biophysical properties (*e.g.*, affinity and/or internalization).

The LOD represents an important parameter that characterizes the analytical performance of a bioassay. In many clinical laboratories and diagnostic applications, LOD is used interchangeably to "sensitivity", "analytical sensitivity" or "detection limit." This may, however, be confusing as the term "sensitivity" is also used in other ways. For example, in some applications "sensitivity" refers to the slope of the calibration curve, which is the definition used by the International Union of Pure and Applied Chemistry (IUPAC).

The LOD is the lowest level of signal of analyte that can be statistically distinguished from a blank sample. In certain conditions, especially to minimize false positive event detection (<0.27%), a higher value of LOD may be chosen and determined as:

$$LOD = Mean_{Blank} + 3\ (SD_{blank})$$

wherein $Mean_{Blank}$ is the mean of blank and "SD" the standard deviation of the measurements.

Mean of blank is determined using a zero calibrator because it is difficult to find samples totally lacking the analyte of interest. The zero calibrator is tested at least 20 times within the same run and the mean and standard deviation of the results are calculated. LOD is considered to be 3 standard deviations above the mean of the blank. Using this formula, the chance of misclassification as positive event compound of interest is 0.27%. Example 1 and Figure 11 show how LOD can be calculated for droplet microfluidics assays using standard curve. In this example, fluorescence signal (in V, y axis) is plot as function of compound concentration (in nM, x axis).

From such calibration plot following, the linear equation f(x) = a x + b can be identified, where f(x) corresponds to the signal measured (e.g. voltage, luminescence, energy, etc.), "b" the value in which the straight line cuts the ordinates axis, "a" the sensitivity of the system (i.e., the slope of the line, or the function relating the measured signal to the quantity to be determined) and "x" the value of the quantity (e.g. temperature, concentration, pH, etc.) to be determined from the signal f(x), the LOD for "x" is calculated as the "x" value in which f(x) equals to the average value of blanks "y" plus "t" times its standard deviation "s" (or, if zero, the standard deviation corresponding to the lowest value measured) where "t" is the chosen confidence value (e.g. for a confidence of 99.3% it can be considered t = 3, determined from the limit of blank). Thus, LOD for x = [f(x)-b]/a = (y + 3 s - b)/a.

In the context of the present invention, the expression "limit of detection" or "LOD" refers to the lowest concentration of an analyte that can be reliably measured by an analytical procedure for a given assay format. Therefore,

as used herein the expression "limit of detection" or "LOD" refers to the "method LOD" or "assay LOD", which does not correspond to the "instrumental LOD". Instrumental LOD can be obtained from the analysis of the analyte in pure solvent. Therefore, "instrumental LOD" only shows the capability of the instrument to detect the analyte and can only be used for comparing different instruments. As used herein, the term "threshold" refers to the "assay LOD" in the context of measuring the biding affinity of the compound of interest and to the critical KD "KDc" in the context of measuring the internalization capacity of the compound of interest. To further discriminate these threshold, the term "first threshold" refers herein to the critical KD "KDc" and the term "second threshold" refers herein to the "assay LOD". Therefore, the "method LOD" or "assay LOD" is used for determining internalization capacity of the compound being able to bind a target, while the "KDc" is used for determining the affinity the compound being able to bind a target. "Method LOD" also takes into account the effects that sample preparation and measurement procedure have on the analysis result. Matched matrix calibration curve methods can be used for determining the LOD, as exemplified in Example 1 and Figure 11.

LOD can be affected by several parameters, as shown in figure 12. While assay reaction can be impacted by assay volume and reaction time, assay LOD is impacted by the quality, the nature and the amount/concentration of tools and reagents used for reading assay reaction. For example, fluorescent based assay LOD can be impacted by fluorescent dyes brightness, and/or extinction coefficient, and/or fluorescence quantum yield. Fluorescence energy transfer assays LOD will depend on molar extinction coefficient, quantum yield, and/or fluorescence half-life.

Since compound, target and/or reporter molecule signaling compound/target reaction are central to perform assay and inform compound/target reaction, assay LOD can be impacted amount of such molecules. In certain circumstances, those molecules can be expressed by cells, in that case very high and/or very low promoter expressing gene can impact assay LOD. Assay LOD depends on the reporter molecule/assay readout (both in terms of the report identity and number).

Assay format can impact as well assay LOD. The use of solid (including, but not limited to particle, beads, slide) support, the number of supports, cell support or performing a reaction in solution can change for the same readout the LOD. In the context of the present invention, the assay LOD is determined by using a beadline assay (see Example 1). However, when referred to the measurement of the internalization capacity, the assay LOD is determined by a cell-based assay.

In the context of the present invention, another parameter used for screening a compound is the critical KD (KDc). KDc depends on the target concentration ($Ag_0$), the assay LOD and the compound concentration ($IgG_0$) and is defined by the following formula:

$$K_D{}^C = \frac{(Ag_0 - LOD)(IgG_0 - LOD)}{LOD}$$

Compound concentration can be estimated using a reporting molecule. At mean/median concentration of the compound, reporting molecule signal will cover most of the signal population (*i.e.*, concentration of compound follows a gaussian distribution - see fig. 14A)

The inventors have surprisingly found that by tuning assay LOD and target concentration ($Ag_0$) it is possible to enrich for specific function (*i.e.*, affinity) and selecting for compound 'signal' being either for the vast majority of cells or below it.

According to one aspect, the present invention relates to a method for identifying and classifying a compound being able to bind a target, in an assay having a limit of detection (LOD) for said compound, the method comprising the steps of:

(a) providing a microreactor comprising a compound and a target,
(b) incubating said compound and said target under conditions sufficient to allow the formation of a complex between said compound and said target,
(c) identifying said complex by means of detecting a signal above or below a first threshold or a second threshold, wherein:

(i) if the signal is below the first threshold, and said target is present in a concentration equal to or up to five-fold greater than said LOD, said compound is classified as having the highest binding affinity for said target;
(ii) if the signal is above the first threshold, and said target is present in a concentration at least five-fold greater than said LOD, said compound is classified as having the lowest binding affinity for said target;
(iii) if the signal is above the second threshold, and said target is present in a concentration lower than said LOD, said compound is classified as exhibiting an internalization capacity,

wherein the limit of detection (LOD) of the compound to be identified and classified ranges from about 0.01 nM to about 10 nM.

As used herein, the indefinite article "a" or "an" may also refer to plural articles, *i.e.*, "one or more" or "at least one". For example, the term "a compound" includes "one or more compound".

As used herein, the term "compound" refers to a molecule capable of binding a specific target. Conversely, the term "target" refers to a molecule capable of being bound by a specific compound. Therefore, as a compound and its target can be defined as binding partners, a molecule acting as a compound can also be a target and *vice versa.*

The compound of interest can be any receptor and the target any ligand, and *vice versa.*

In one embodiment, the compound is selected from an antibody, a ligand and a receptor.

In one embodiment, the compound or target is an antibody.

As used herein, the term "antibody" refers to an immunoglobulin molecule having immunological reactivity with a specific antigen, or a protein molecule serving as a receptor for specifically recognizing an antigen (*e.g.*, T cell receptor (TCR)-like antibodies). Accordingly, the term "antibody" includes monoclonal antibody, bispecific antibody, antibody-drug conjugate (ADC), engineered antibody, nanobodies, polyclonal antibody, recombinant antibody, whole antibody (antibody consisting of at least two heavy chains and two light chains linked by disulfide bonds) and antibody fragments, such as single-chain variable fragment (ScFv), fragment antigen binding (Fab) and F(ab')2, Fc-fusion proteins. The whole antibody includes IgA, IgD, IgE, IgM and IgG, the latter comprising the subclasses IgG1, IgG2, IgG3, and IgG4. Non-limiting examples of IgG subclasses are: Human IgG subclasses: IgG1, IgG2, IgG3, and IgG4 - some of them if not all are kept in transgenic animals; mouse IgG subclasses: IgG1, IgG2a, IgG2b, IgG3; rat IgG subclasses: IgG1, IgG2a, IgG2b, IgG2c; rabbit and goat IgG.

While information on the concentration of a compound to be identified and classified might be known, e.g., from the supplier, the method disclosed herein does not require the determination of the ideal concentration of the compound for its binding with the target.

In one embodiment, the compound is provided in the microreactor in a non-determined concentration.

It is understood by a person skilled in the art that concentration depends on the amount of material (mass or number of molecule) and volume for performing an assay. The higher the volume is, the lower the concentration will be for a fixed number of molecules. In the present examples, concentration are provided in nM. In the present examples, assay reactions are performed in a volume of 80 pL.

In the context of the present invention, the ligand can be for example T cell antigen (from a TCR / T cell antigen recognition pair), B cell antigen (from a B cell receptor / B cell antigen recognition pair), stimulatory immune checkpoint molecule (*e.g.*, OX40 from an OX40L / OX40 pair), inhibitory immune checkpoint molecule (*e.g.*, PD-1 from a PD-L1 / PD-1 pair), cytokines (from a cytokine / cytokine receptor pair), carbohydrates (from a selectin / carbohydrate pair), members of the immunoglobulin superfamily (from a pair comprising two members of the immunoglobulin superfamily), selectin (from a member of the immunoglobulin superfamily / selectin pair), chemokines (from a chemokine / chemokine receptor pair), hormones (from an hormone / hormone receptor pair), growth factors (from a growth factor / growth factor receptor pair), ligands of GPCRs (from a GPCR / corresponding ligand pair) or substrates (from an enzyme / corresponding substrate pair). In some embodiment, the set of ligands is a set of T cell antigens (peptides, glycolipids or small metabolites such as 5-A-RU derivatives), preferably bound to major histocompatibility complex (MHC molecules that can be class I, class II or MR1) or to CD1a, b, c, or d molecules.

In one embodiment, the ligand is selected from T cell antigen, B cell antigen, stimulatory immune checkpoint molecule, inhibitory immune checkpoint molecule, cytokines, carbohydrates, members of the immunoglobulin superfamily, selectin, chemokines, hormones, growth factors, ligands of GPCRs or substrates.

In one embodiment, the compound or target is a cell receptor.

In another embodiment, the compound or target is a T-cell receptor.

In the context of the present invention, the receptor can be for example T cell receptor (TCR, from a TCR / T cell antigen recognition pair), B cell receptor (from a B cell receptor / B cell antigen recognition pair), receptor for stimulatory immune checkpoint molecules (e.g., OX40L from an OX40L / OX40 pair), receptor for inhibitory immune checkpoint molecules (*e.g.*, PD-L1 from a PD-L1 / PD-1 pair), cytokine receptor (from a cytokine / cytokine receptor pair), selectin (from a selectin / carbohydrate pair), integrin (from an integrin / member of the immunoglobulin superfamily pair), member of the immunoglobulin superfamily (from a member of the immunoglobulin superfamily / selectin pair, or from a pair comprising two members of the immunoglobulin superfamily), cadherin (from a pair comprising two cadherins), chemokine receptor (from a chemokine / chemokine receptor pair), hormone receptor (from an hormone / hormone receptor pair), growth factor receptor (from a growth factor / growth factor receptor pair), G protein-coupled receptor (GPCR, from a GPCR / corresponding ligand pair), chimeric antigen receptor or enzyme (from an enzyme / corresponding substrate pair).

In one embodiment, the receptor is selected from T cell, B cell receptor, receptor for stimulatory immune checkpoint molecules, receptor for inhibitory immune checkpoint molecules, cytokine receptor, selectin, integrin, member of the immunoglobulin superfamily, cadherin, chemokine receptor, hormone receptor, growth factor receptor, G protein-coupled receptor or enzyme.

In the context of the present invention, the compound and/or the target can be provided directly or indirectly into the microreactor. A direct provision of the compound and/or the target refers to the event when the compound and/or the target is present in the microreactor at a predefined concentration. An indirect provision of the compound and/or the target refers to the event when the compound and/or the target is generated in the microreactor following a chemical reaction or incubation. A non-limiting example of an indirect provision of the compound and/or the target is the provision of an antibody secreting cell that following incubation generates antibodies within a range of concentration. Another example of an indirect provision of the compound and/or the target is the provision of the same in the form of a precursor that following a chemical reaction generates the compound and/or the target within a range of concentration.

In one embodiment, the compound is provided in the microreactor in a concentration ranging from about 0.1 nM to about 100 nM, preferably about 0.5 nM to about 50 nM.

In another embodiment, the target is provided in the microreactor in a concentration ranging from about 0.1 nM to about 75 nM, preferably about 1 nM to about 30 nM.

In the context of the present invention, the term "about" is used herein to mean "near to", "close to". When the term "about" is used in conjunction with a specific value or a numerical range, it modifies that value or range by extending the boundaries above and below the numerical values set forth. As used herein, the term "about" modifies a numerical value above and below the stated value by a variance of 10 percent, up or down (higher or lower).

As used herein, the term "assay" refers to assays whereby a compound-target complex is formed and/or detected. Such assays include, but are not limited to, Western blotting, immunoprecipitation, immunofluorescence, immunocytochemistry, immunohistochemistry, fluorescence activated cell sorting (FACS), fluorescence in situ hybridization (FISH), immunomagnetic assays, ELISA, ELISPOT, agglutination assays, aggregation assays, solubility assays, sandwich immune-assays, cell-based assays, cell-cell interaction assays, flocculation assays, cell panning and the like. Suitable assays for carrying out the method according to the claimed invention are described hereinafter.

As used herein, the term "cell" refers to eukaryote and/or procaryote cells, primary, engineered and/or synthetic cells.

The LOD might vary according to the assay selected, due to, for example, selected fluorophore, particle/cell size, detection method. Therefore, the LOD of an assay for detecting a target bound to a solid support might not correspond to the LOD of an assay for detecting the same target exposed on a single cell surface.

Typically, the LOD for many assays is determined using serial blank measures to describe measurement error. All assays include a number of possible sources of reproducibility and/or measurement error. With antibody-based assays, pipetting errors, incomplete mixing of samples and/or reagents, contamination, non-specific binding, and random instrument processes may contribute to both inconsistency and inaccuracy. The possible sources of test variability and/or measurement error include sample preparation, contamination and random instrument processes. The "instrumental LOD" includes contamination arising from reagents, but not the sample preparation process. "Method LOD" includes contamination arising from reagents and the sample preparation process.

The LOD of an assay for carrying out the method according to the present invention ranges from 0.01 nM to 10 nM. Preferably, from 0.1 nM to 8 nM. More preferably, from 0.1 nM to 5 nM. Ideally, the assay has a LOD of 1 nM.

The KDc of an assay for carrying out the method according to the present invention ranges from 0.001 nM to 1000 nM. Preferably, from 0.01 nM to 100 nM. More preferably, from 0.1 nM to 10 nM. Ideally, the assay has a KDc of 5 nM.

The binding affinities of ligands for GPCRs typically falls in the nanomolar (nM) to micromolar ($\mu$M) range. Some small molecule drugs can have binding affinities in the low picomolar (pM) range. Enzyme-linked receptors (including receptor tyrosine kinases) often have binding affinities for their ligands in the nanomolar (nM) to picomolar (pM) range. Nuclear receptors, which respond to small, lipophilic hormones, typically have binding affinities in the nanomolar (nM) range. The binding affinities of ligands (including neurotransmitters and drugs) for ion channels can vary widely, from micromolar ($\mu$M) to picomolar (pM) concentrations.

In the context of the present invention, when the compound is an antibody, the expression "high affinity" refers to an antibody having a $K_D$ of < 10 nM, preferably < 1 nM. When the compound is a TCR, the expression "high affinity" refers to a TCR-pMCH having a $K_D$ of < 10 $\mu$M, ideally below 1 $\mu$M. When the compound is a ligand-GPCR, the expression "high affinity" refers to a ligand-GPCR having a $K_D$ of < 5 nM, ideally < 1 nM.

In the context of the present invention, when the compound is an antibody, the expression "medium affinity" refers to an antibody having a 50 nM > $K_D$ of > 10 nM. When the compound is a TCR, the expression "medium affinity" refers to a TCR-pMCH having a 100 $\mu$M > $K_D$ of > 10 $\mu$M. When the compound is a ligand-GPCR, the expression "medium affinity" refers to a ligand-GPCR having a 50 nM > $K_D$ of > 5 nM.

In the context of the present invention, when the compound is an antibody, the expression "low affinity" refers to an antibody having a $K_D$ of > 50 nM. When the compound is a TCR, the expression "low affinity" refers to a TCR-pMCH having a $K_D$ of > 100 $\mu$M. When the compound is a ligand-GPCR, the expression "low affinity" refers to a ligand-GPCR having a $K_D$ of > 50 nM.

In the context of the present invention, the target can be expressed by, or displayed on, the surface of a cell (or cells), synthetic cell(s), a bead, and/or engineered APC-like beads as disclosed in Neal *et al.* (Neal et al. J. Immunol. Res.

Ther. 2017, 2:68-79), or *in vitro* encoded, as disclosed in Grubaugh *et al.* (Grubaugh et al. Vaccine 2013, 31:3805-3810).

In one embodiment, the target is either bound to a solid support or exposed on a single cell surface.

As used herein, the term "solid support" refers to any non-biological matrix (e.g., magnetic beads, gel matrix/beads or affinity matrix/beads) that has a given specificity for a target molecule such that the target molecule can be immobilized on said support, which allows isolation of the target molecule from the content comprised in the microreactor.

In another embodiment, the target is a macromolecule selected from protein, peptide, carbohydrate, lipid and nucleic acid.

In the context of the present invention, the term "microreactor" refers to a container for performing chemical or physical processes within a series of fluidly connected modules. The microreactor is characterized by at least one cross-sectional dimension, such as depth, width, length, diameter, etc. ranging from about 0.001 nanometer to about $15 \times 10^6$ nanometer. Examples of fluidly connected modules are chambers, channels, reservoirs or the like. A non-limiting example of microreactor is a microfluidic droplet within a microfluidic device.

In one embodiment, the microreactor is selected from a droplet, a well, a chamber and a tube.

As used herein, the term "droplet" refers to an isolated portion of a fluid which is immiscible with its surrounding. In the context of the present invention, the "droplet" may be spherical, substantially spherical or non-spherical in shape. The shape may depend by different parameters, such as, for example, the external environment.

As used herein, the terms "well", "chamber" and "tube" refers to any structure in which volumes of fluid can be contained. These structures can have different volume dimensions (pico-, nano-, micro-, millimeter).

In another embodiment, the microreactor has a volume ranging from about $10^{-6}$ $\mu$L to about 2000 $\mu$L, preferably from about 0,000001 $\mu$L to about 1 $\mu$L, more preferably from about 0,000001 $\mu$L to about 0,01 $\mu$L.

In the context of the present invention, the incubation step is intended either for allowing a compound to be produced in suitable amount for being detected and for allowing a compound to bind its target, thereby forming a complex.

Incubation conditions can vary depending on the assay intended to be carried out or the concentration of the compound and/or target under investigation.

When assaying an antibody, the incubation step is an important aspect as it allows antibody binding its antigen. Suitable incubation conditions must be determined carefully for each assay. Antibody concentrations that are too high and incubation times that are too long can result in an unspecific background signal. Conversely, concentrations that are too low and incubation times that are too short can lead to a very weak or missing signal. An increased incubation time can increase the time available for an antibody to find and bind its antigen, which is important when using low-affinity antibodies or when antibodies have a limited access to the antigens.

In the context of the present invention, the incubation parameters are suitable for efficient detection of both surface and intracellular protein of interest (*e.g.*, intracellular cytokines, transcription factors).

Typical eucaryote cells based and compound related incubation temperature ranges from 4 °C to 37 °C +/- 2 °C, procaryote/virus/archae cell based and compound related incubation temperature ranges from 4 °C up to > 95 °C. Typical incubation time goes from milliseconds (for kinetics assay) to more than 24h (for cell-cell interaction mediated compound production regulation assay).

In one embodiment, the incubation step is carried out in at least one microreactor for about 1 to about 24 hours.

In the context of the present invention, the identification step allows the detection of the formation of the complex between the compound and its target.

In the context of the present invention, the signal above a threshold to be detected can originate from a direct detection of the complex between at least one compound and at least one target, or as a consequence of an indirect detection of the complex (*i.e.*, secondary detection agent, indirect coupling, or induction of secretion of molecule, intra cellular signaling, and similar).

In one embodiment, the identification step is carried out by means of fluorescence readout, luminescence readout or phosphorescence readout. A signal is emitted as consequence of either a chemical or enzymatic reaction.

As used herein, the term "fluorescence readout" includes but is not limited to direct fluorescence and indirect fluorescence (*e.g.*, FRAP, FLIP, FRET, BRET, FLIM, PRIM).

In one embodiment, the compound with the highest binding affinity for a target is enriched when the concentration of the target is at most 5 nM and signal is below a first threshold.

In another embodiment, the compound with the lowest biding affinity for a target is enriched when the concentration of the target of up to 15 nM and signal is above a first threshold.

In the context of the present invention, the selection of an assay having an LOD of 1 nM, and the provision of a target in a concentration ranging from about 5 nM to about 15 nM and a compound in a concentration ranging from about 1 nM to about 10 nM, allows the method disclosed herein to enrich a compound having a specific biological function.

In one embodiment, if the LOD of the assay is about 1 nM, and the compound is present in a concentration ranging from about 1 nM to about 5 nM, and

i. the target is present in a concentration of about 5 nM, detected complexes are enriched for the highest binding affinity for said target;
ii. the target is present in a concentration of about 15 nM, detected complexes are enriched for the lowest binding affinity for said target.

In one embodiment, if the LOD of the assay is about 1 nM, the signaling molecule is present at a concentration below LOD and the target is present at any concentration, the compound is classified as exhibiting an internalization capacity when signal is detected above a second threshold.
According to another aspect, the present invention relates to a system for screening a compound in an assay having a predetermined limit of detection (LOD), the system comprising:

(a) a first module configured for collecting a compound and a target;
(b) a second module configured for incubating said compound and said target;
(c) a third module configured for detecting an interaction between said compound and said target;
(d) a fourth module configured for screening said compound.

In the context of the present invention, the first module is directly or indirectly fluidically connected to the second module, which is directly or indirectly fluidically connected to the third module, which is directly or indirectly fluidically connected to the forth modules. The LOD in connection with the first and second module refers to a "method LOD". The LOD in connection with the third and fourth refers to an "instrumental LOD". The third module is configured to detect a signal above a threshold, i.e., the formation of a complex between the at least one compound and the at least one target. Said threshold may refers to a first threshold or a second threshold. Depending on the nature of the signal to be detected, suitable further module can be selected accordingly. For example, a fluorimeter can be adopted for measuring parameters of spectrum fluorescence (300 - 1000 nm).
Embodiments and definitions of the methods disclosed herein also apply to the system disclosed herein.

## Examples

### *Example 1*

### *Determining LOD assay using beadline format*

To determine assay LOD in a given system, the assay is performed with conditions mimicking the real assay. In the case of the beadline assay, the same reagents (*i.e.*, para magnetic beads, assay buffer, and reporting molecule) shall be used/similar to the condition used for the corresponding screening assay. Here example is shown for a beadline assay where molecule of interest is detected with DL550 dye. Other detecting method and dyes can be used. The paramagnetic beads being coupled with Streptavidin, reporting molecule (DL550) is attached indirectly to beads via a biotinylated oligo, such that a single DL550 molecule is bound to a single oligo. The number of biotin sites per beadline per container exceeds the number of biotin oligo incubated/used for LOD calculation.
To determine the minimal sensitivity of the assay, different condition of molecule/dye per beadline was tested:

1 - 0 nM oligos DL550
2 - 1 nM oligos DL550
3-5 nM oligos DL550
4-25 nM oligos DL550
5 - 125 nM oligos DL550

Assay buffer was prepared as follow (final concentration):

- RPMI without phenol red
- Nycodenz (23 %)
- Low IgG serum (5 %)
- HEPES (25 mM)
- Pluronic F-68 (0.1 %)

The paramagnetic beads mix were prepared as follows:

| | Final concentration (in droplets) |
|---|---|
| AdemTech Streptavidin plus, 300 nm | 16.5 % |
| Oligo-DL550-biotin | 0 - 1 nM - 5 nM - 25 nM - 125 nM |

The beads were pre-treated as described in Gérard et al., Nat. Biotechnol. 2020, 38(6):715-721. In brief:

Taking 33 µL of beads for each 100 µL of coflow1 solution.
Putting on magnet (Thermo MagJet rack 12x1.5) 1 min, resuspend beads in PBS at 33 %.
Washing with PBS, and put on magnet 1 min.
Discarding supernatant and repeat PBS wash.
Resuspending beads in 10 % pluronic F127 (20% beads).
Sonicating 15 min on ice (sonicator VWR USC-TH).
Putting on magnet 4 min, and resuspend in PBS.
Splitting in 5 tubes of 400 µL.

Oligo solutions were prepared as 100X solutions, add 4uL of labelled oligonucleotide per 400uL bead tube. Then performing serial 5-fold dilution to reach desired final concentration.
Adding oligos to the beads with instant mixing.
Incubating 30min at RT.
Putting on magnet 1min and discard supernatant. Resuspending in 200uL PBS for each 100uL of mix.
Incubating 5min at RT
Repeating PBS wash
Resuspending beads at 33% in assay buffer.
Generating 80 pL droplets using state of the art water in oil emulsion droplet generator. The 5 different conditions can be distinguished one from another by using different concentrations of another fluorescent dye (here Dy754).
The prepared emulsion is analyzed on a droplet reader, as described in Gérard et al., Nat. Biotechnol. 2020, 38(6):715-721. Defining the gates for all the 5 different populations and recording the data for all the individual frequency one by one.
Figure 11 reports signal intensities for each concentration of reporting dye and LOD calculation.

***Example 2***

***Definition of LOD in cell-based assay with particle or cell.***

To determine assay LOD in a given system, the assay is performed with conditions mimicking the real assay. In the case of the cell-based assay, the same reagents (*i.e.*, reporter cell, assay buffer, and reporting molecule) shall be used/similar to the condition used for the corresponding screening assay. Here example is shown for a cell-based assay where molecule of interest is detected with DL550 dye. Other detecting method and dyes can be used. The reporter cells expressing target of interest, reporting molecule (DL550) will bind to compound of interest binding to said target indirectly. The number of targets expressed at the surface of the reporter cell may vary from one reporter cell-based assay and from one cell to another. In the later example, the expression variability preferably do not exceed a 10-fold.
To assess assay LOD, while ensuring specificity, different conditions are run:

- condition 1: reporter cell in the presence of the detection molecule but without compound of interest
- condition 2: reporter cell in the presence of the detection molecule and 1 nM of compound of interest
- condition 3: reporter cell in the presence of the detection molecule and 10 nM of compound of interest

Assay is performed in the following assay buffer

| Reagents (final concentration) | Quantity for 1 mL (µL) |
|---|---|
| RPMI without phenol red | 635 |
| Nycodenz (23 %) | 230 |

(continued)

| Reagents (final concentration) | Quantity for 1 mL (μL) |
|---|---|
| Low IgG serum (5 %) | 50 |
| HEPES-KOH (25 mM) | 25 |
| Pluronic F-68 (0.1 %) | 10 |
| Nucgreen | 50 |

Filtering the assay buffer using a syringe and 0.45μm hydrophobic filter (Cil, ref SF13PT45C).

Cells are prepared following the protocol below:

- Counting reporter cells,
- Spinning 5min 400 g and resuspend cells at 1 million per mL in warm PBS (Phosphate Buffer Saline) + CTV (Cell Trace Violet, 1:4000),
- Incubating 20 min at 37 °C,
- Adding 5x volume of warm RPMIc (complete RPMI media) and incubate 5 min at 37°C,
- Washing twice in cold PBS,
- Filtering cells through cell strainer (70 μm),
- Counting cells again,
- Preparing reagent solution#1: assay buffer + detection molecule at 12.5 nM final concentration in the droplet,
- Spinning and resuspend cells at 7.5 million per mL in reagent solution#1.

Solution containing primary antibody binding to reporter cell is prepared, containing either 0 nM, 1 nM or 10 nM.

To distinguish the different conditions used reporter dye called 'Dropcode DY754' (Dyomics - 754-00, reference E10-10032) is added together in the primary Ab solution at 0.2 μM, 0.6 μM or 1.8 μM final concentration in the droplets.

80 pL droplets containing cells and reagents according to Gérard et al., Nat. Biotechnol. 2020, 38(6):715-721 were generated.

Droplets are collected and incubated at 4 °C for 1 h to allow primary antibody, detection molecule complex binding to reporter cell.

Binding of primary antibody specific to target cell is monitored and detected via relocalization of the detection molecule onto target cell when primary antibody is present. Droplet fluorescent analysis is performed on a droplet scanner, as per Gérard et al., Nat. Biotechnol. 2020, 38(6):715-721 and on a static droplet analyser DropMap, as per Eyer et al., Nat. Biotech 2017, 35(10):977-982. More than 10,000 droplets are analyzed in each condition.

Assay LOD is the concentration of primary antibody for which relocalization of primary antibody onto reporter cells is equivalent to signal observed in the absence of primary antibody.

Typical results are shown in Figure 13. Results show that Assay LOD is about 1 nM using imaging readout and 0.7 nM using droplet analyzer.

***Example 3***

***Running assay for high affinity Ab***

1 - PBMCs thawing

Thawing PBMCs vials in 50 mL of prewarmed complete medium, then pelleting cells at 400 g for 5 min at 4 °C. Suspend cells in 10 mL warm PBS and count cells.

2 - PBMCs CTV staining

Suspending cells at 1 million per mL in warm PBS + CTV (1:4000) and incubating 20 min at 37 °C. Adding 5x volume of warm RPMIc (minimum) and incubating 5 min at 37 °C. Spinning cells and washing with warm RPMlc, then washing 2 times at 4 °C in ice cold PBS and filtering through cell strainer (70 μm). Counting cells.

Optionally PBMC can be further purified in B cells, memory B cells and memory B cells can further be activated.

4 - Performing the beadline assay

4-1 - Preparing assay buffer

| Reagents (final concentration) | Quantity for 1 mL (μL) |
|---|---|
| RPMI without phenol red | 635 |
| Nycodenz (23 %) | 230 |
| Low IgG serum (5 %) | 50 |
| HEPES-KOH (25 mM) | 25 |
| Pluronic F-68 (0.1 %) | 10 |
| Nucgreen | 50 |

Filter the assay buffer using a syringe and 0.45 μm hydrophobic filter (Cil, ref SF13PT45C)

| Reagent | Full description | Stock concentration | Final concentration (in droplets) |
|---|---|---|---|
| Beads | AdamTech Streptavidin plus, 300 nm | 100% | 16.5 % |
| Capture antibody | biotin anti-human IgG CH1 | 1 mg / mL = 76.9 μM | 1000 nM |
| Detection Ab (reagent sol#1) | Goat anti-human IgG DL550 | 0.5 mg / mL = 3.3 μM | 12.5 nM |
| Antigen (reagent sol#1) | antigen-DL650 | 1.4 mg / mL =9.3 μM | 5 nM |

| | | | |
|---|---|---|---|
| Dropcode (reagent sol#1) | DY754 | 100 μM | 0.4 μM |

4-2 - Preparing magnetic beads (=coflow#1)

- Vortexing beads stock for 10 sec.
- Taking 33 μL of beads for each 100 μL of coflow1 solution.
- Putting on magnet (Thermo MagJet rack 12x1.5) 1 min, resuspend beads in PBS at 33 % (100 μL for each 100 μL of coflow#1 solution).
- Washing with PBS (no incubation), and put on magnet 1 min. Discard supernatant and repeat PBS wash.
- Resuspending beads in 10 % pluronic F127 (20% beads).
- Sonicating 15 min on ice (sonicator VWR USC-TH).
- Putting on magnet 3-4 min, and resuspend in PBS (16.5 % beads, 200 μL for each 100uL of coflow#1 solution).
- Adding capture Ab to the beads with instant mixing and incubate 30 min at RT.
- Putting on magnet 1 min, discard supernatant.
- Washing with assay buffer, 5 min (use 200 μL for each 100 μL of coflow#1 solution). Putting on magnet 2 min. Discarding supernatant. Repeating assay buffer wash.
- Resuspending beads at 33 % in reagent solution#1 (assay buffer + detection antibody + antigen).

4-3 - Preparing cells/Ab (=coflow#2)

- Resuspending CTV-stained cells in assay buffer at 7.5 million per mL here.
- Adding DY754 to account for dropcode.

4-4 - Droplet generation

- Generating 80 pL droplet, similar to Gérard et al., Nature Biotechnology 2020, 38(6):715-721.
- Collecting tube (2mL tube) was kept on round magnet and on ice all along of droplet generation, incubation, and droplet reinjection.

4-5 - Cell secretion

In order to let the cells secrete antibody to the desired concentration, and knowing it is expected them to secrete a mean of 5 nM (median 2 nM) of antibody per hour in 80 pL droplet, the collection tube was kept 90 min at 37 °C.

4-6 - Signal analysis

Droplet were either injected in droplet analyser (at 200Hz, 1kHz and 2kHz) and into a chamber for imaging.
High affinity antibodies have a detectable signal above threshold for at least antigen fluorescent molecules.

***Example 4***

***Running assay for medium/low affinity Ab***

Similar to example 3, except that mix assay buffer solution (step 4-1) is as follow:

| Reagent | Full description | Stock concentration | Final concentration (in droplets) |
|---|---|---|---|
| Beads | AdamTech Streptavidin plus, 300 nm | 100% | 16.5 % |
| Capture antibody | biotin anti-human IgG CH1 | 1 mg / mL = 76.9 μM | 1000 nM |
| Detection Ab (reagent sol#1) | Goat anti-human IgG DL550 | 0.5 mg / mL = 3.3 μM | 12.5 nM |
| Antigen (reagent sol#1) | antigen-DL650 | 1.4 mg / mL =9.3 μM | 30 nM |
| Dropcode (reagent sol#1) | DY754 | 100 μM | 0.4 μM |

Medium/low affinity antibodies have a detectable signal above threshold for at least antigen fluorescent molecules.

***Example 5***

***Running assay for internalizing Ab***

Similarly to example 1, where assay LOD determination is performed, two primary antibodies are used at different concentration (ranging from LOD to above LOD): one having properties of being internalized in reporter cell, the other not. To identify internalizing antibodies, either the detection molecule concentration is calibrated to show not detectable signal when non internalizing antibody is used and show detectable signal when internalizing is used or the reporter cell is expressing target below assay LOD, which is only detectable when antibody is internalized.

***Example 6***

***Screening library of ligand and receptor***

Similarly to example 2, two cell types are encapsulated in 80 pL droplets: one producing ligand library and the second producing receptor library. Alternatively, one ligand can be screened against a library of receptor. Conversely, one type of receptor can be screened against a library of ligand.
Receptor, receptor library, ligand and/or ligand library can as well be present as cell free molecules, like in solution, on particle, on aggregates of particles and the like.

Interaction of ligand and receptor is monitored via assay using signalling molecule as readout (secreted molecule, fluorescent molecule expression, internalization, downstream signalling pathway activation), either directly or indirectly.
Detection of the interaction can be selected based on assay LOD, which can be tuned to detect only the desired events having a selected binding affinity. In this example assay LOD is LOD of detection of secreted molecule, fluorescent molecule expression, internalization, downstream signalling pathway activation, which can be performed either directly or indirectly.
To select desired assay LOD, it is conceivable to have (1) brighter fluorescent dyes, (2) very high and/or very low promoter expressing gene, and/or (3) high or low abundance signalling fluorescent molecule.

***Example 7***

***Selecting assay format based on desired antibody function***

Using similar experimental conditions as set in figure 15, it is possible to selectively enrich for antibodies having specific functions. It is estimated, when screening 1 millions of enriched B cells, with secretion rate and assay condition similar to Eyer et al., Nature 2017 (*i.e.*, primary B cells from spleen or bone marrow), when critical KD (KDc) is either ~ 10 nM (upper part of the table) and ~ 20 nM (lower part of the table), it is possible to specifically enrich for antibodies having KD < KDc by selecting the antigen concentration ($Ag_0$) and assay having a selected LOD.
In brief, assay conditions are described in example 3, especially assay buffer composition, beads preparations, and droplet generation and incubation, as well as droplet fluorescence signal reading.

***Example 8***

***Selecting antibody having specific function***

Using similar experimental conditions as set in figure 16, it is possible to selectively enrich for antibodies having specific functions. In this example, secretion rate of primary B cells isolated from immunized animal, is ~ 5 nM (per 80 pL droplet after 1h incubation). When cells are prepared as per Eyer et al., Nature Biotech 2017 or Gérard et al., Nature Biotech 2020, and droplets are prepared as per example 3, it is conceivable to detect both antigen binding the secreted antibody and amount of secreted molecule by using antibody detection molecule. At mean secretion, the vast majority of cells will show a similar antibody detection molecule signal intensity. Based on this information, and working in assay condition having desired LOD (here 1 nM) and antigen concentration ($Ag_0$), the critical KD (KDc) detected at mean secretion is identified (and indicated in Figure 16). In those conditions it is possible to enrich specifically antibodies having affinity below KDc based on assay LOD, antigen concentration $Ag_0$ and secretion signal at or below the mean of cell secretion (without knowing the exact value of it).

**Claims**

1. Method for identifying and classifying a compound being able to bind a target, in an assay having a limit of detection (LOD) for said compound, the method comprising the steps of:

   (a) providing a microreactor comprising a compound and a target,
   (b) incubating said compound and said target under conditions sufficient to allow the formation of a complex between said compound and said target,
   (c) identifying said complex by means of detecting a signal above or below a first threshold or a second threshold, wherein:

      (i) if the signal is below the first threshold, and said target is present in a concentration equal to or up to five-fold greater than said LOD, said compound is classified as having the highest binding affinity for said target;
      (ii) if the signal is above the first threshold, and said target is present in a concentration at least five-fold greater than said LOD, said compound is classified as having the lowest binding affinity for said target;
      (iii) if the signal is above the second threshold, and said target is present in a concentration lower than said LOD, said compound is classified as exhibiting an internalization capacity,

   wherein the limit of detection (LOD) of the compound to be identified and classified ranges from about 0.01 nM to about 10 nM.

2. The method according to claim 1, wherein the compound is provided in a non-determined concentration.

3. The method according to any of the claims 1 and 2, wherein said at least one target is provided in the microreactor in a concentration ranging from about 0.1 nM to about 75 nM.

4. The method according to any of the claims 1 to 3, wherein said at least one compound is provided in the microreactor with a concentration ranging from about 0.1 nM to about 100 nM.

5. The method according to any of the claims 1 to 4, wherein said at least one target is either bound to a solid support or exposed on a single cell surface.

6. The method according to any of the claims 1 to 5, wherein said at least one target is a macromolecule selected from the group consisting of protein, peptide, carbohydrate, lipid and nucleic acid.

7. The method according to any of the claims 1 to 6, wherein said at least one compound is selected from an antibody, a ligand and a receptor.

8. The method according to claim 7, wherein the ligand is selected from T cell antigen, B cell antigen, stimulatory immune checkpoint molecule, inhibitory immune checkpoint molecule, cytokines, carbohydrates, members of the immunoglobulin superfamily, selectin, chemokines, hormones, growth factors, ligands of GPCRs or substrates.

9. The method according to claim 7, wherein the receptor is selected from T cell, B cell receptor, receptor for stimulatory immune checkpoint molecules, receptor for inhibitory immune checkpoint molecules, cytokine receptor, selectin, integrin, member of the immunoglobulin superfamily, cadherin, chemokine receptor, hormone receptor, growth factor receptor, G protein-coupled receptor or enzyme.

10. The method according to any of the claims 1 to 9, wherein said at least one microreactor is selected from a droplet, a well, a chamber and a tube.

11. The method according to any of the claims 1 to 10, wherein said at least one microreactor has a volume ranging from $10^{-6}$ μL to 2000 μL.

12. The method according to any of the claims 1 to 11, wherein the incubation step is carried out in said at least one microreactor for about 1 to 24 hours.

13. The method according to any of the claims 1 to 12, wherein the identification step is carried out by means of fluorescence readout, luminescence readout or phosphorescence readout.

14. System for screening a compound in an assay having a predetermined limit of detection (LOD), the system comprising:

(a) a first module configured for collecting a compound and a target;
(b) a second module configured for incubating said compound and said target;
(c) a third module configured for detecting an interaction between said a compound and said target;
(d) a fourth module configured for screening said compound.

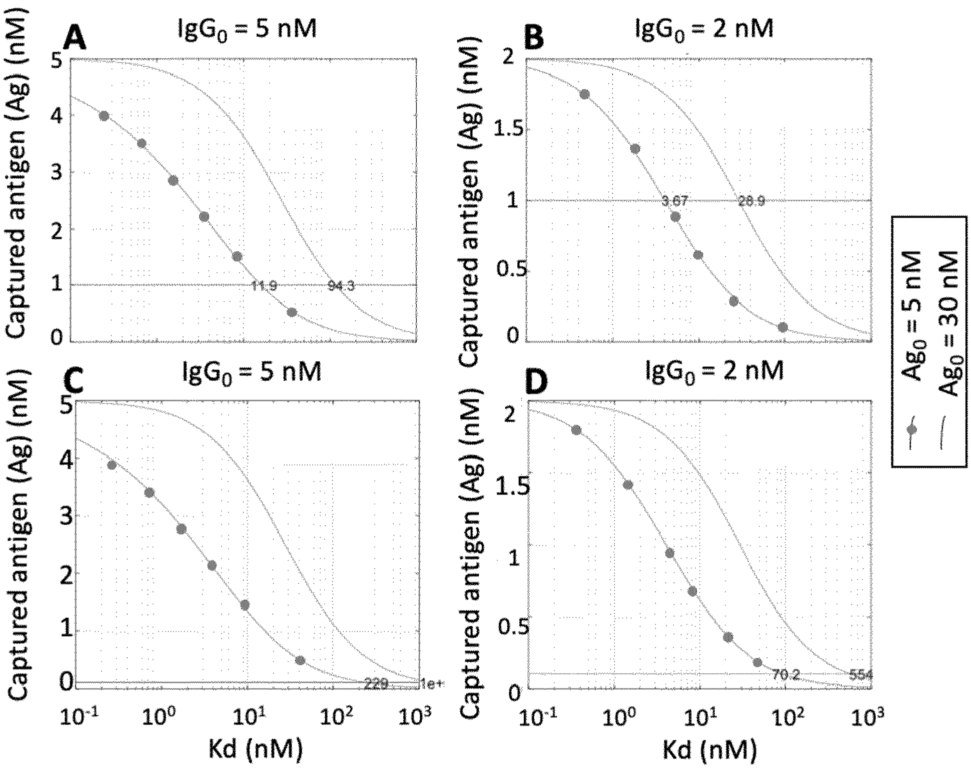
A
IgG0 = 5 nM
B
IgG0 = 2 nM
C
IgG0 = 5 nM
D
IgG0 = 2 nM
Captured antigen (Ag) (nM)
Kd (nM)
11.9
94.3
3.67
28.9
229
1e+
70.2
554
Ag0 = 5 nM
Ag0 = 30 nM

Figure 1A-D

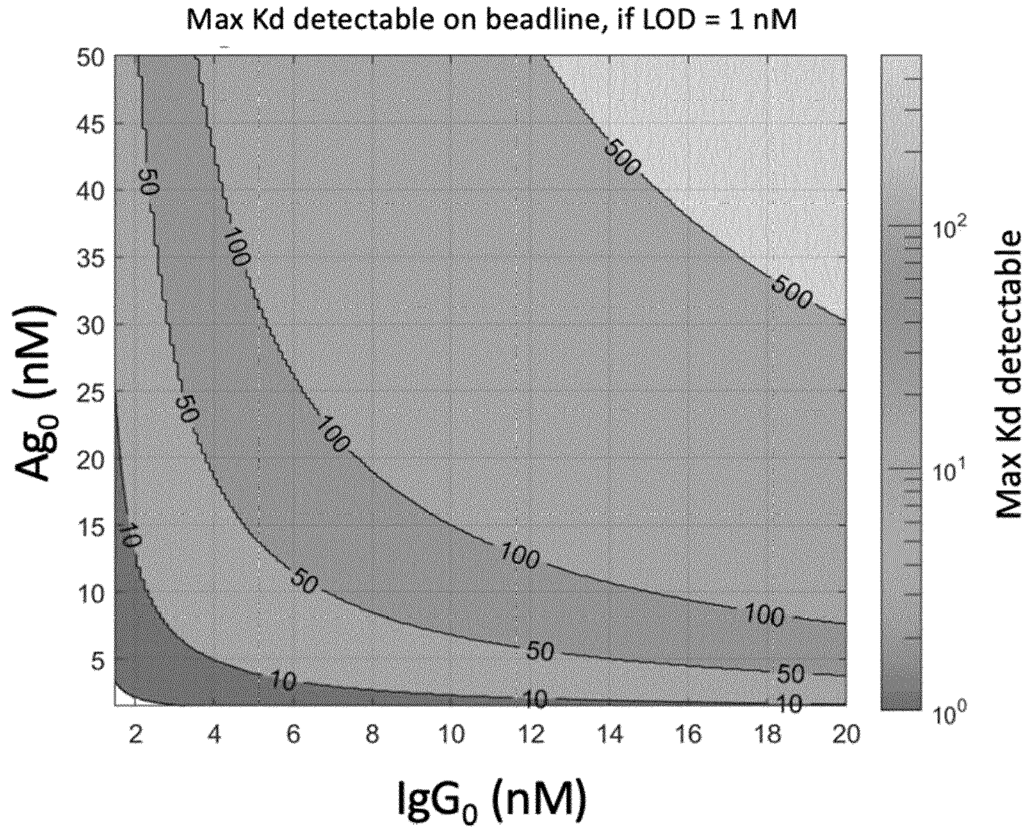
Max Kd detectable on beadline, if LOD = 1 nM
50
45
40
35
30
25
20
15
10
5
2
4
6
8
10
12
14
16
18
20
10
50
100
500
$Ag_0$ (nM)
$IgG_0$ (nM)
$10^2$
$10^1$
$10^0$
Max Kd detectable

Figure 2

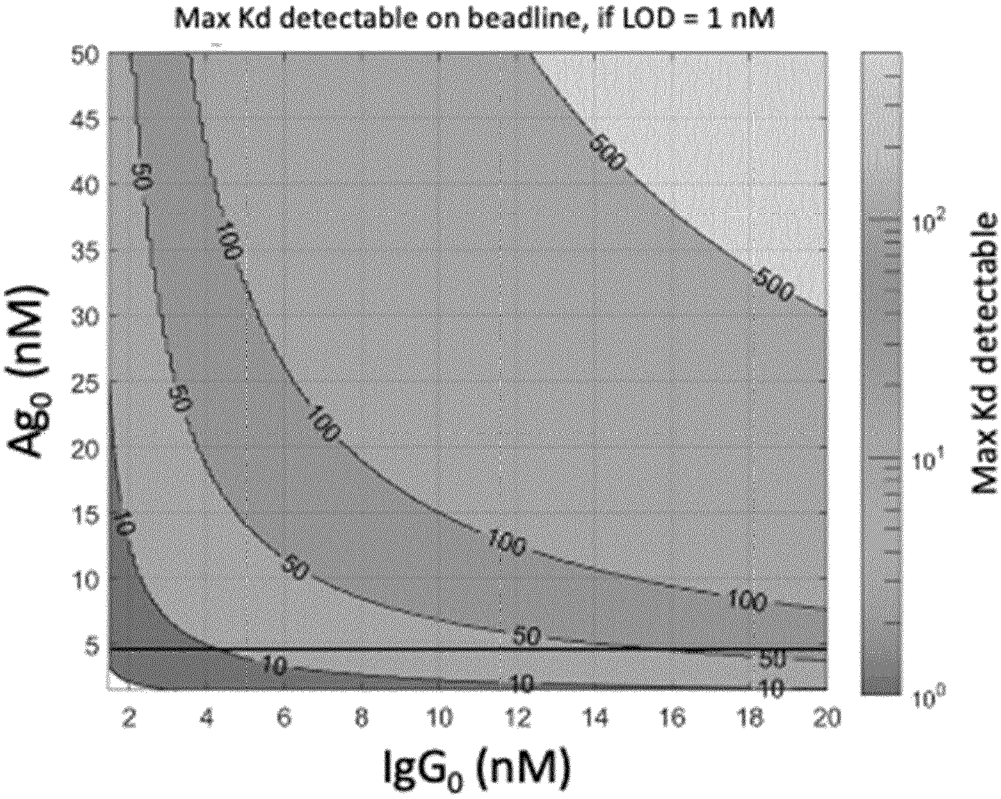
Max Kd detectable on beadline, if LOD = 1 nM
50
45
40
35
30
25
20
15
10
5
Ag0 (nM)
2
4
6
8
10
12
14
16
18
20
IgG0 (nM)
500
100
50
10
$10^2$
$10^1$
$10^0$
Max Kd detectable

Figure 3

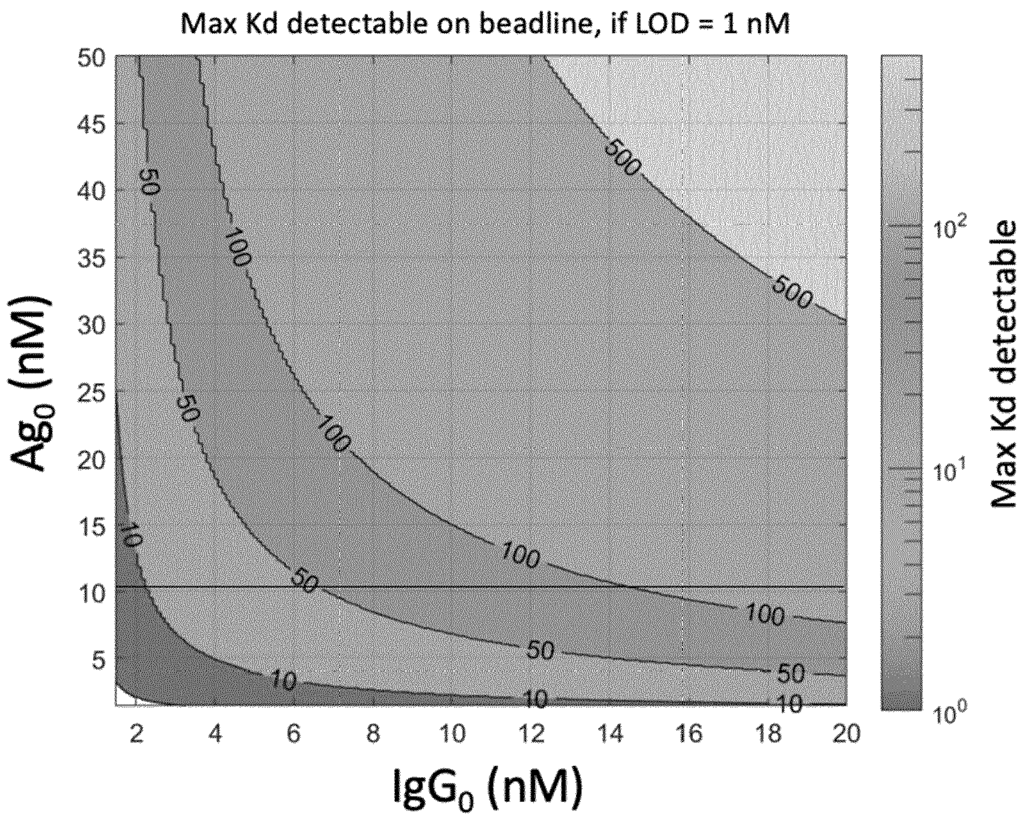
Max Kd detectable on beadline, if LOD = 1 nM
50
45
40
35
30
25
20
15
10
5
$Ag_0$ (nM)
2
4
6
8
10
12
14
16
18
20
$IgG_0$ (nM)
500
100
50
10
$10^2$
$10^1$
$10^0$
Max Kd detectable

Figure 4

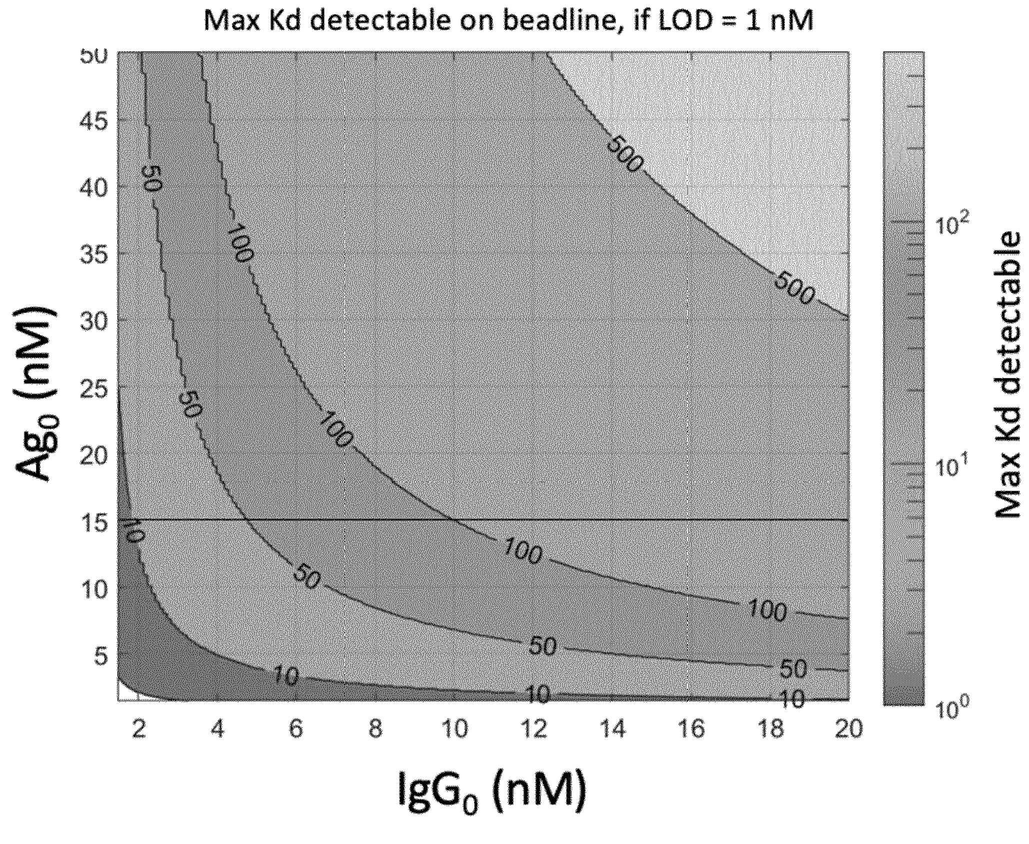
Max Kd detectable on beadline, if LOD = 1 nM
Ag0 (nM)
IgG0 (nM)
Max Kd detectable
50
45
40
35
30
25
20
15
10
5
2
4
6
8
10
12
14
16
18
20
500
100
50
10
10^2
10^1
10^0

Figure 5

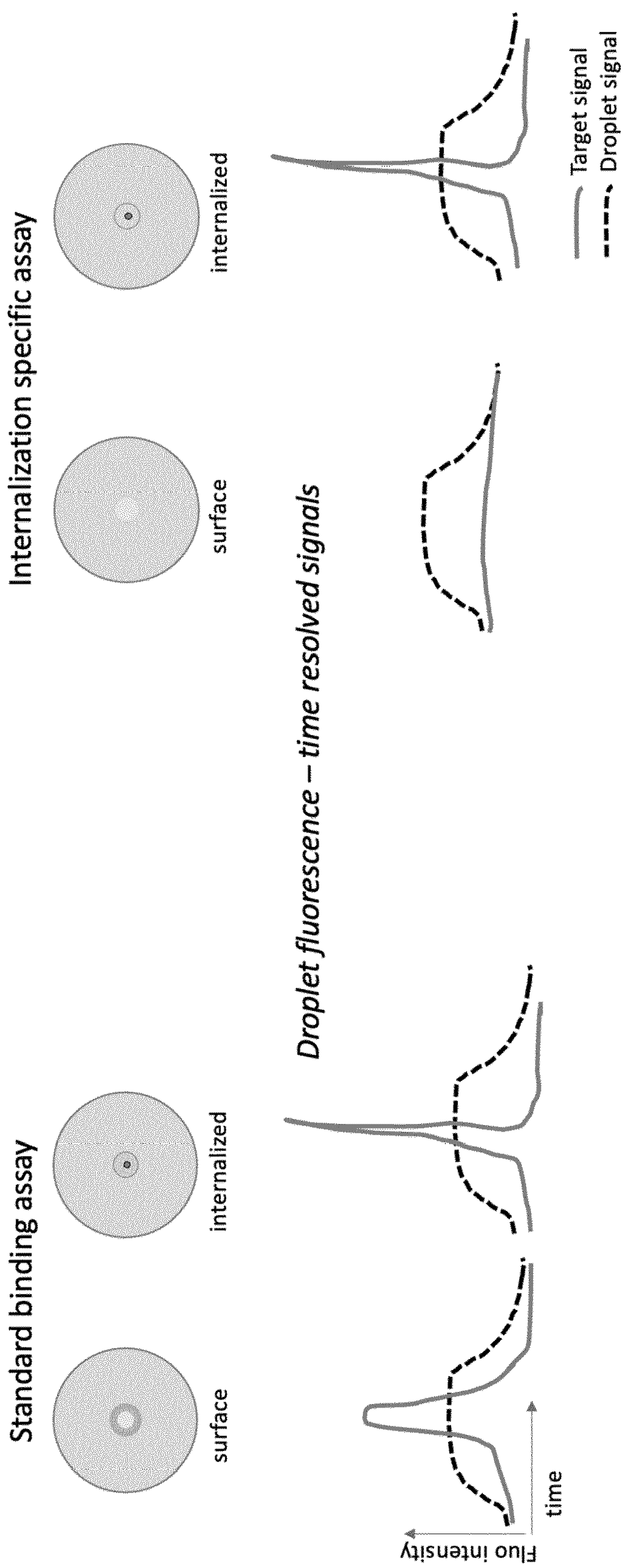
Standard binding assay
Internalization specific assay
surface
internalized
surface
internalized
Droplet fluorescence – time resolved signals
Fluo intensity
time
Target signal
Droplet signal

Figure 6

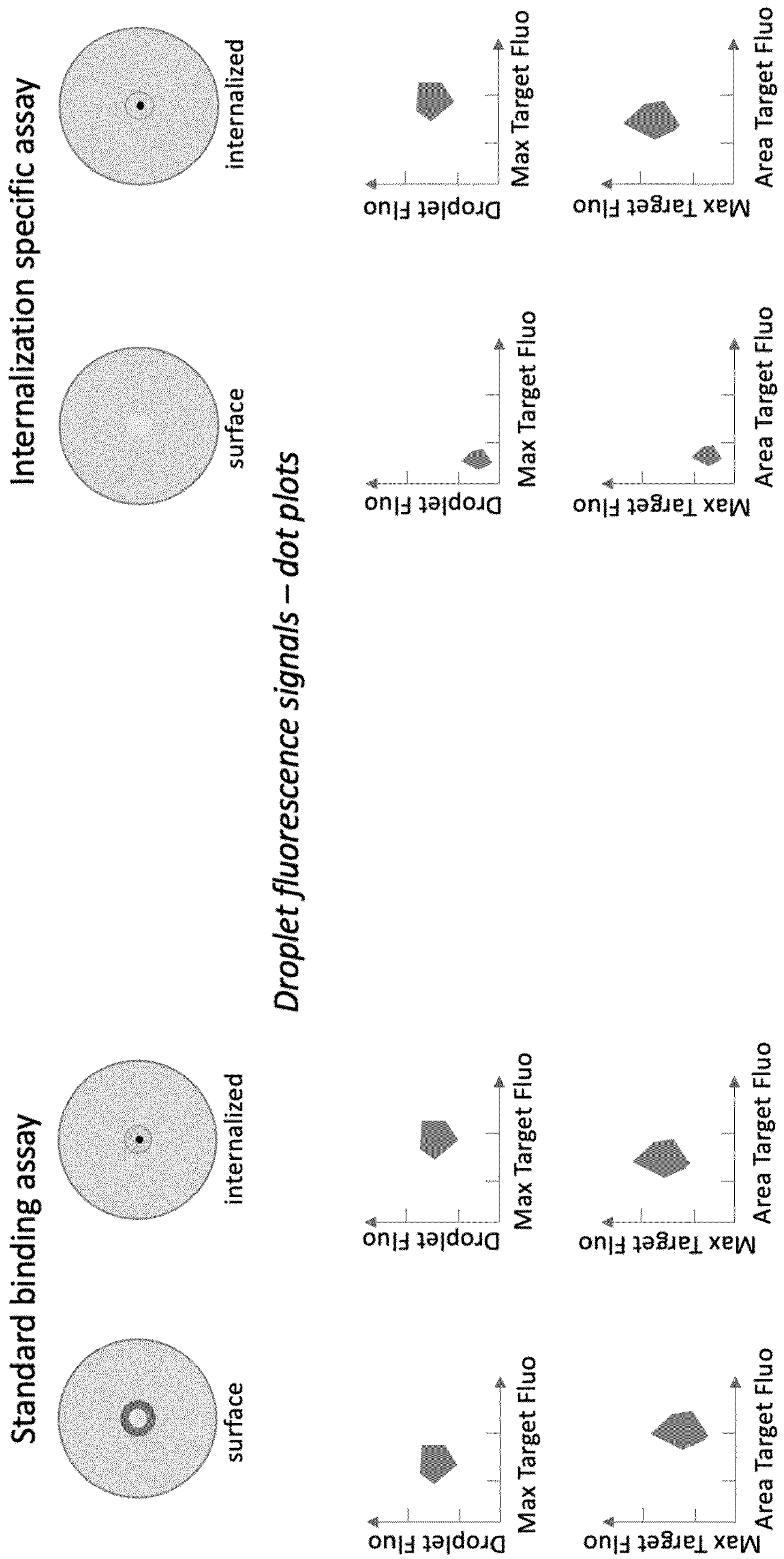
Standard binding assay
surface
internalized
Droplet Fluo
Max Target Fluo
Max Target Fluo
Area Target Fluo
Droplet fluorescence signals – dot plots
Internalization specific assay
surface
internalized
Droplet Fluo
Max Target Fluo
Max Target Fluo
Area Target Fluo

Figure 7

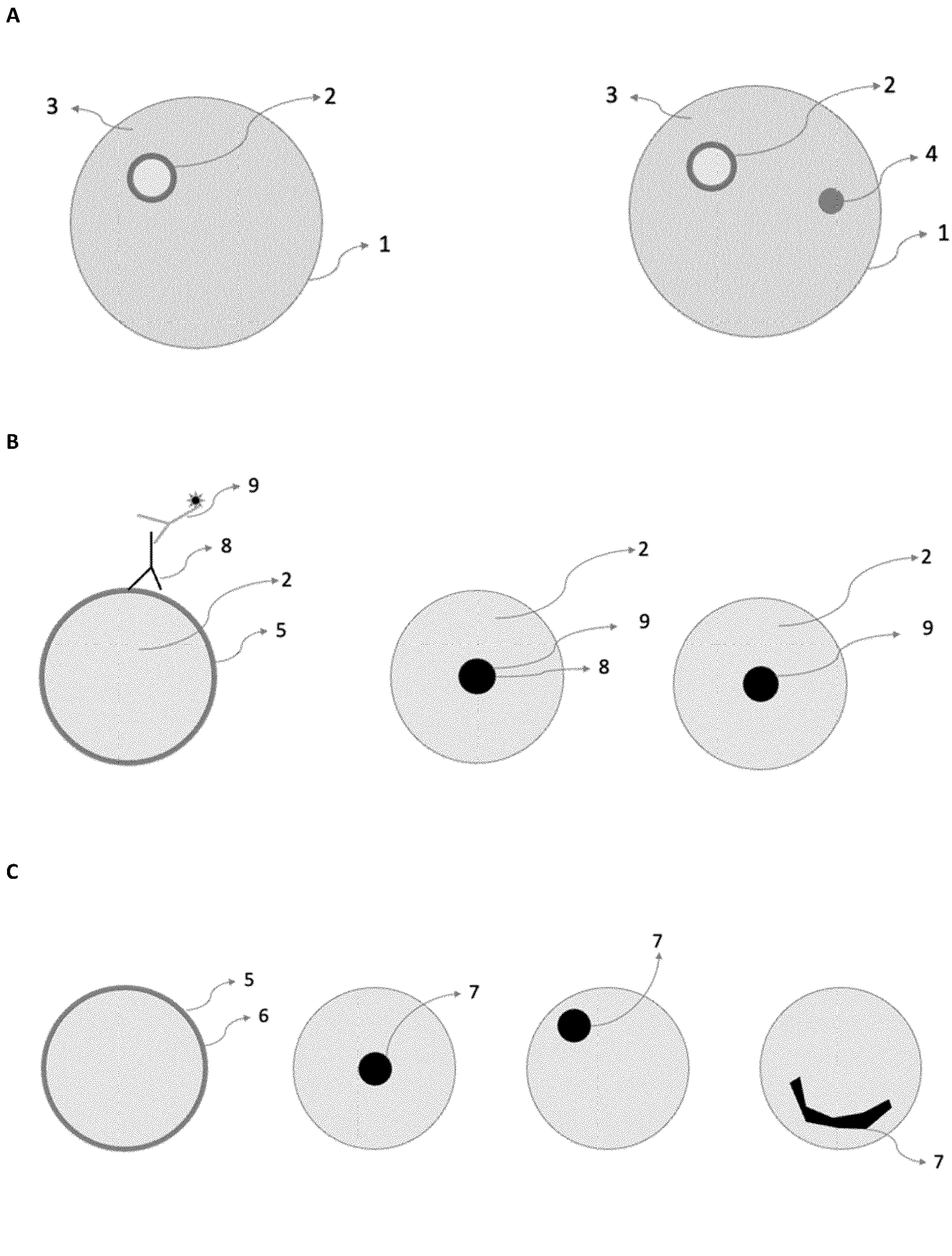


Figure 8A-C

A

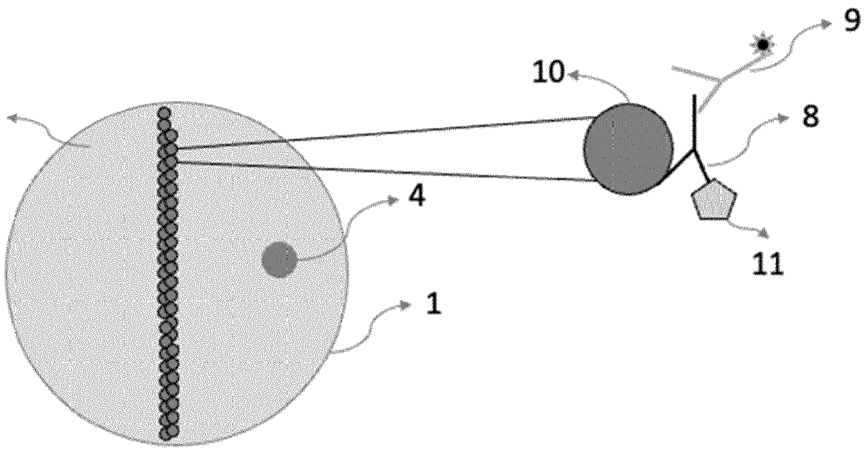


B

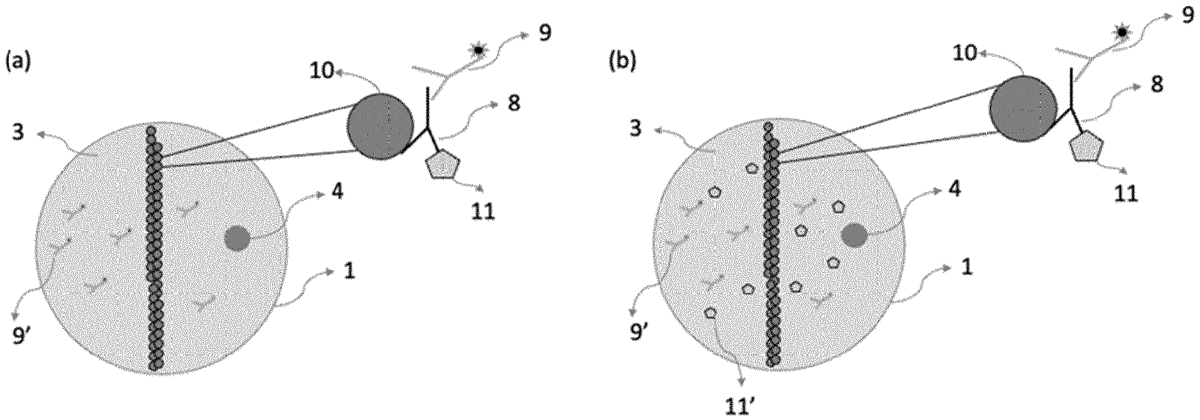


Figure 9A-B

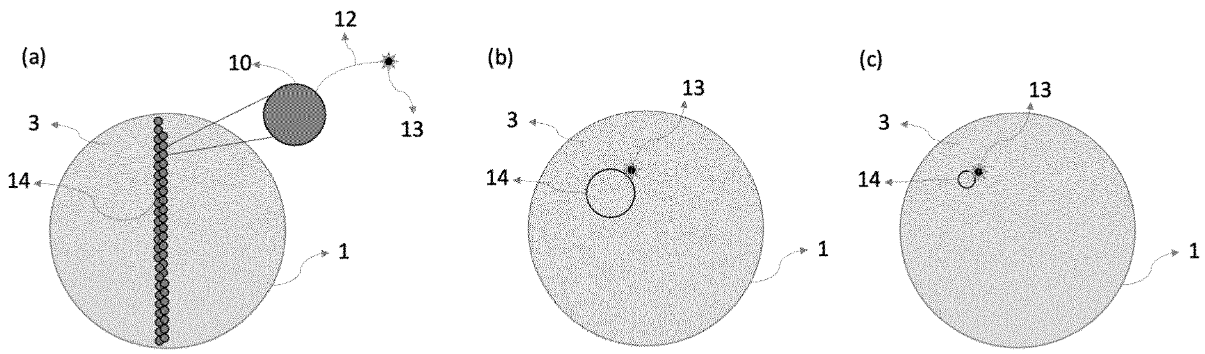


Figure 10A-C

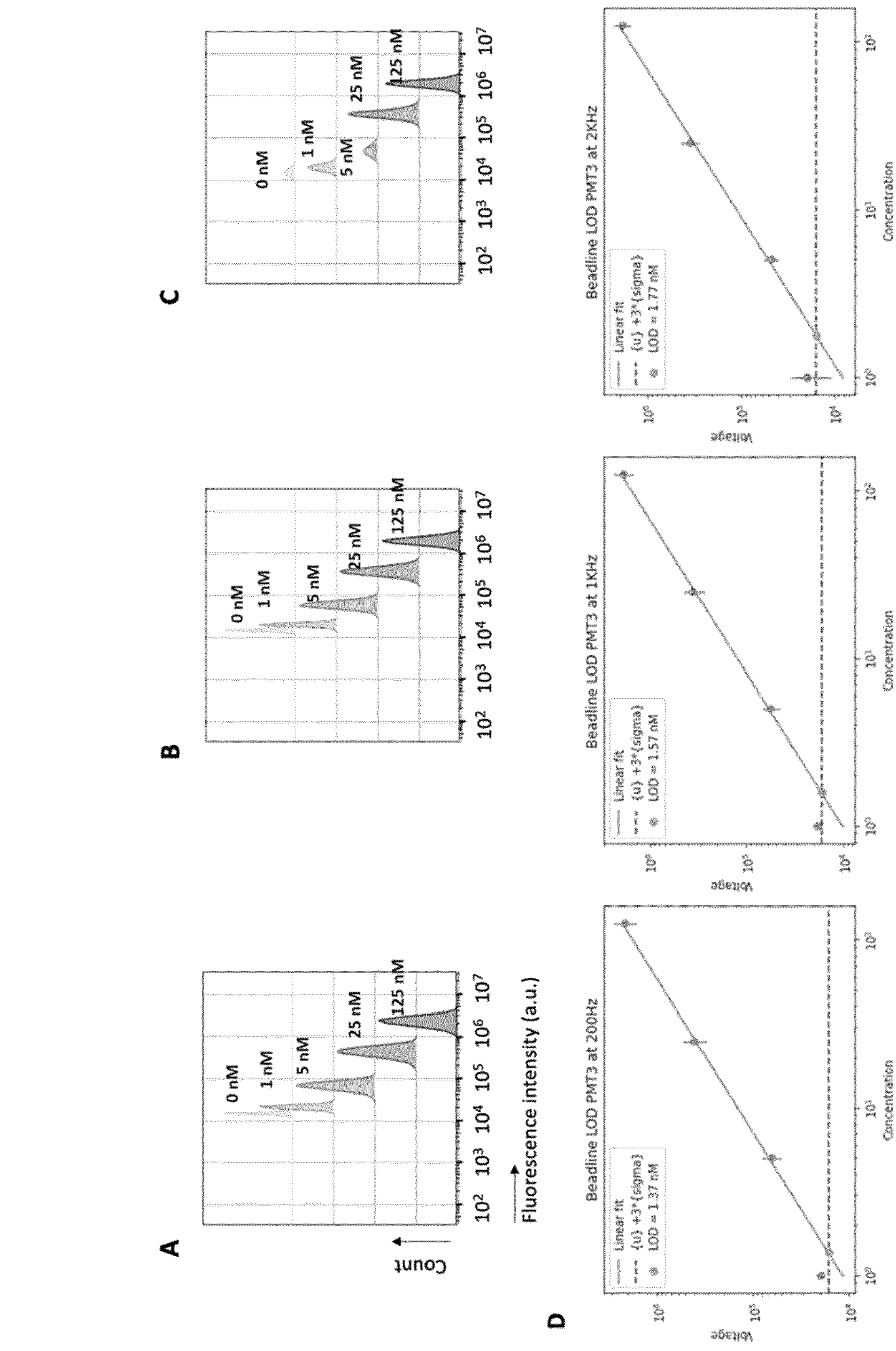
A
B
C
D
0 nM
1 nM
5 nM
25 nM
125 nM
Count
Fluorescence intensity (a.u.)
Beadline LOD PMT3 at 200Hz
Beadline LOD PMT3 at 1KHz
Beadline LOD PMT3 at 2KHz
Linear fit
{u} +3*{sigma}
LOD = 1.37 nM
LOD = 1.57 nM
LOD = 1.77 nM
Voltage
Concentration

Figure 11A-D

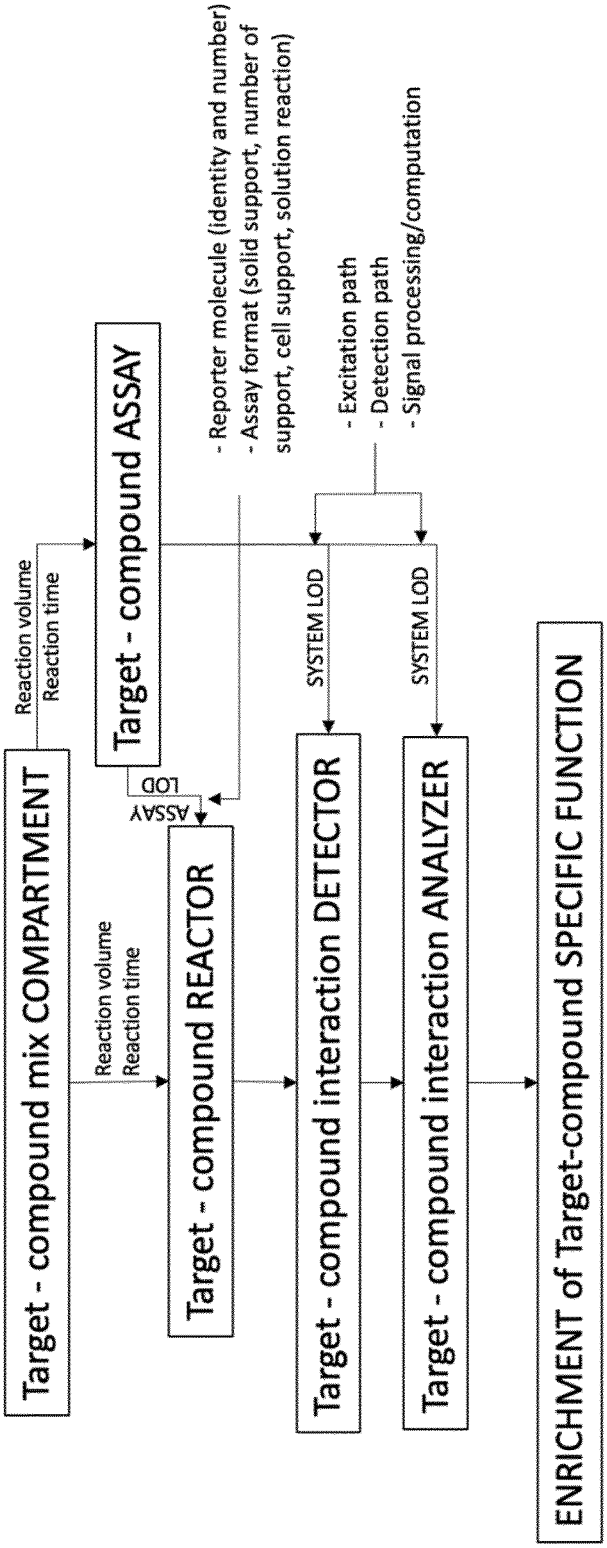
Target - compound mix COMPARTMENT
Reaction volume
Reaction time
Target - compound ASSAY
Reaction volume
Reaction time
ASSAY LOD
- Reporter molecule (identity and number)
- Assay format (solid support, number of support, cell support, solution reaction)
Target - compound REACTOR
SYSTEM LOD
- Excitation path
- Detection path
- Signal processing/computation
Target - compound interaction DETECTOR
SYSTEM LOD
Target - compound interaction ANALYZER
ENRICHMENT of Target-compound SPECIFIC FUNCTION

Figure 12

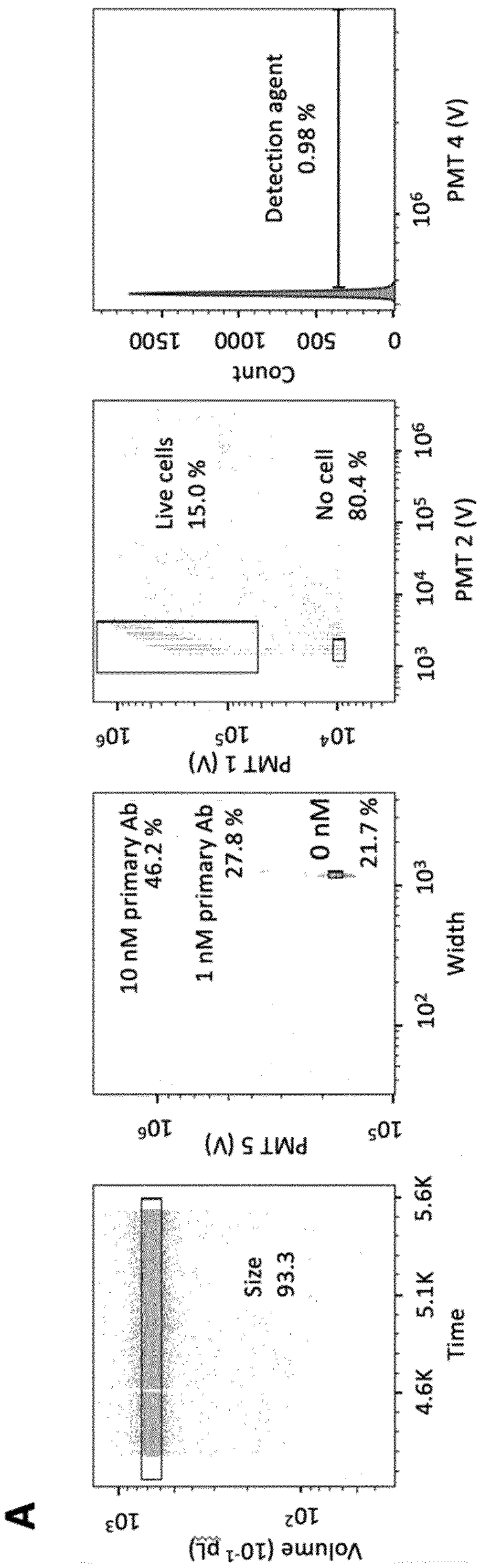
A
Volume (10-1 pL)
10^3
10^2
Time
4.6K
5.1K
5.6K
Size
93.3
PMT 5 (V)
10^6
10^5
Width
10^2
10^3
10 nM primary Ab
46.2 %
1 nM primary Ab
27.8 %
0 nM
21.7 %
PMT 1 (V)
10^6
10^5
10^4
PMT 2 (V)
10^3
10^4
10^5
10^6
Live cells
15.0 %
No cell
80.4 %
Count
1500
1000
500
0
PMT 4 (V)
10^6
Detection agent
0.98 %

Figure 13A

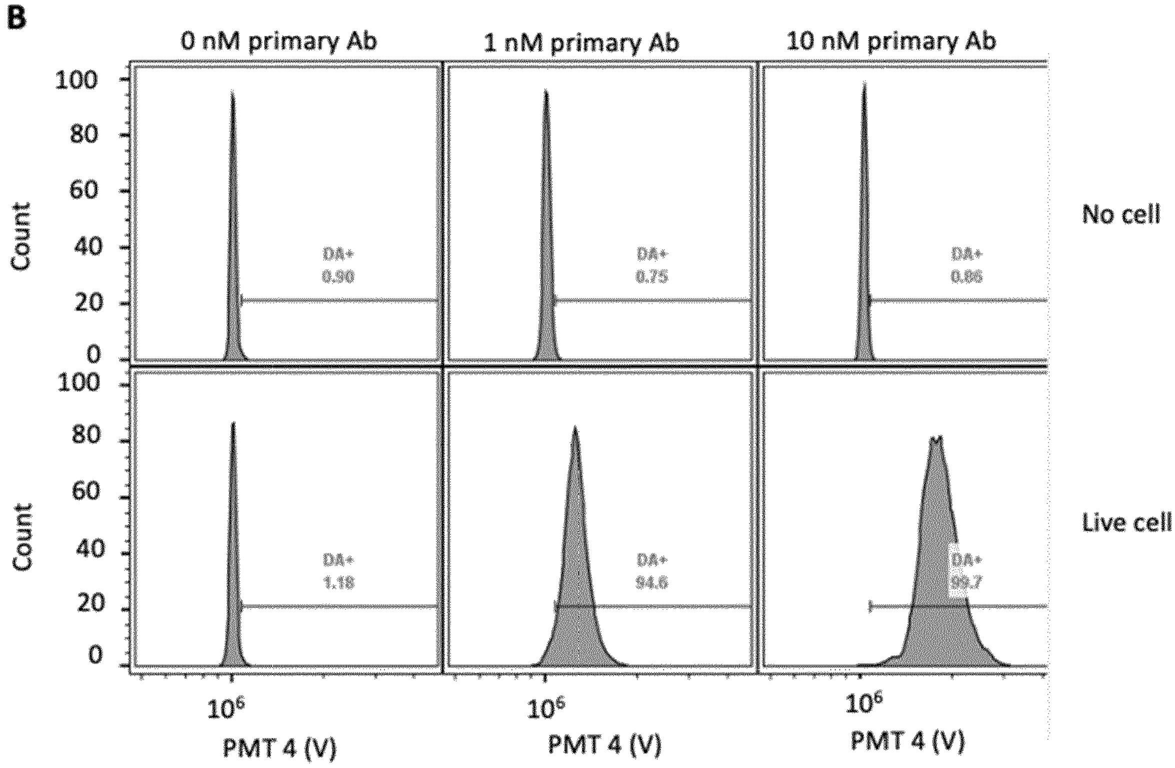
B
0 nM primary Ab
1 nM primary Ab
10 nM primary Ab
Count
100
80
60
40
20
0
DA+
0.90
0.75
0.86
1.18
94.6
99.7
No cell
Live cell
$10^6$
PMT 4 (V)

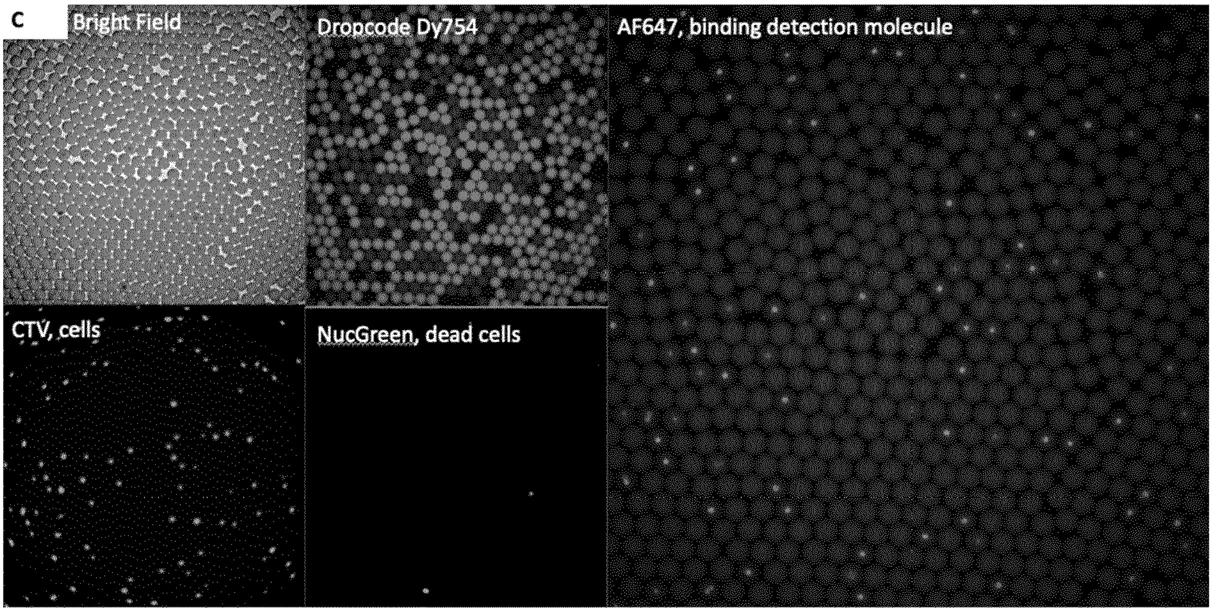
C
Bright Field
Dropcode Dy754
AF647, binding detection molecule
CTV, cells
NucGreen, dead cells

Figure 13B-C

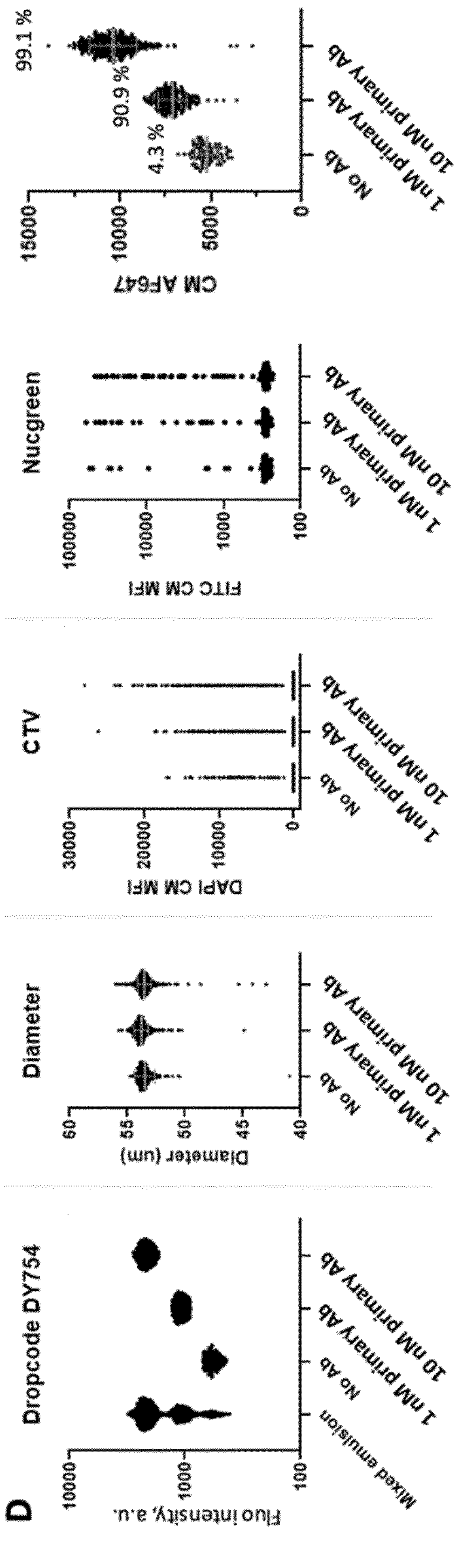
D
Dropcode DY754
Fluo intensity, a.u.
10000
1000
100
Mixed emulsion
No Ab
1 nM primary Ab
10 nM primary Ab
Diameter
Diameter (um)
60
55
50
45
40
CTV
DAPI CM MFI
30000
20000
10000
0
Nucgreen
FITC CM MFI
100000
10000
1000
100
CM AF647
15000
10000
5000
0
4.3 %
90.9 %
99.1 %

Figure 13D

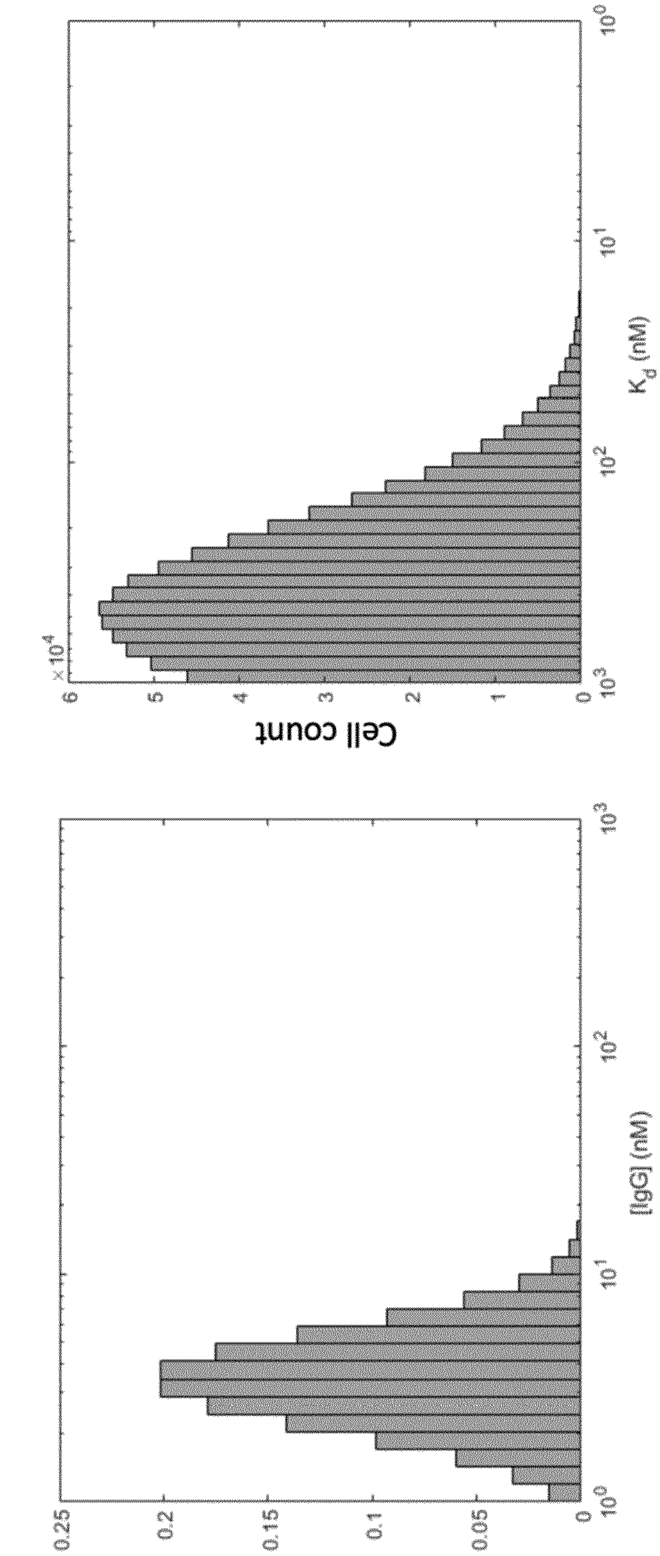
A
Frequency
0
0.05
0.1
0.15
0.2
0.25
$10^0$
$10^1$
$10^2$
$10^3$
[IgG] (nM)
Cell count
$\times10^4$
0
1
2
3
4
5
6
$10^3$
$10^2$
$10^1$
$10^0$
$K_d$ (nM)

Figure 14A

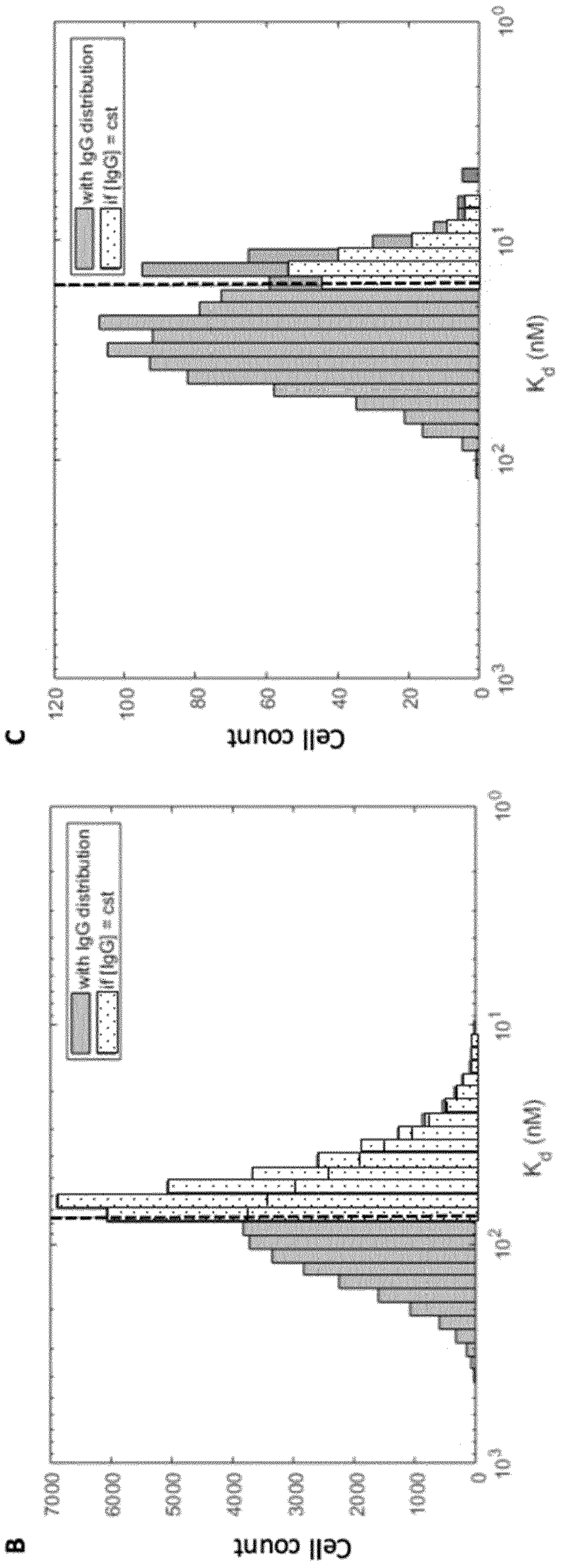


Figure 14B-C

| Critical KD (KDc) (nM) | Ag0 (nM) | LOD (nM) | Estimated number of cells having KD < KDc * | Estimated number of sorted cells having KD < KDc * | Estimated total number of sorted cells * | Efficiency (%) | Purity (%) |
|---|---|---|---|---|---|---|---|
| 9 | 1,51 | 0,5 | 46 | 22 | 129,3 | 47,8 | 17,0 |
| 10 | 3,5 | 1 | 46 | 24,5 | 216,5 | 53,3 | 11,3 |
| 10 | 8,67 | 2 | 46 | 22,9 | 365 | 49,8 | 6,3 |
| 10 | 18 | 3 | 46 | 21,1 | 802 | 45,9 | 2,6 |
| 19 | 2,62 | 0,5 | 46 | 40,4 | 1356 | 87,8 | 3,0 |
| 20 | 6 | 1 | 46 | 38,6 | 1780 | 83,9 | 2,2 |
| 20 | 15,3 | 2 | 46 | 34 | 2577 | 73,9 | 1,3 |
| 20 | 33 | 3 | 46 | 28 | 4688 | 60,9 | 0,6 |

*** Estimated from 1 million screened cells, with secretion rate and assay condition as per Eyer et al., Nature Biotechnology 2017**

Figure 15

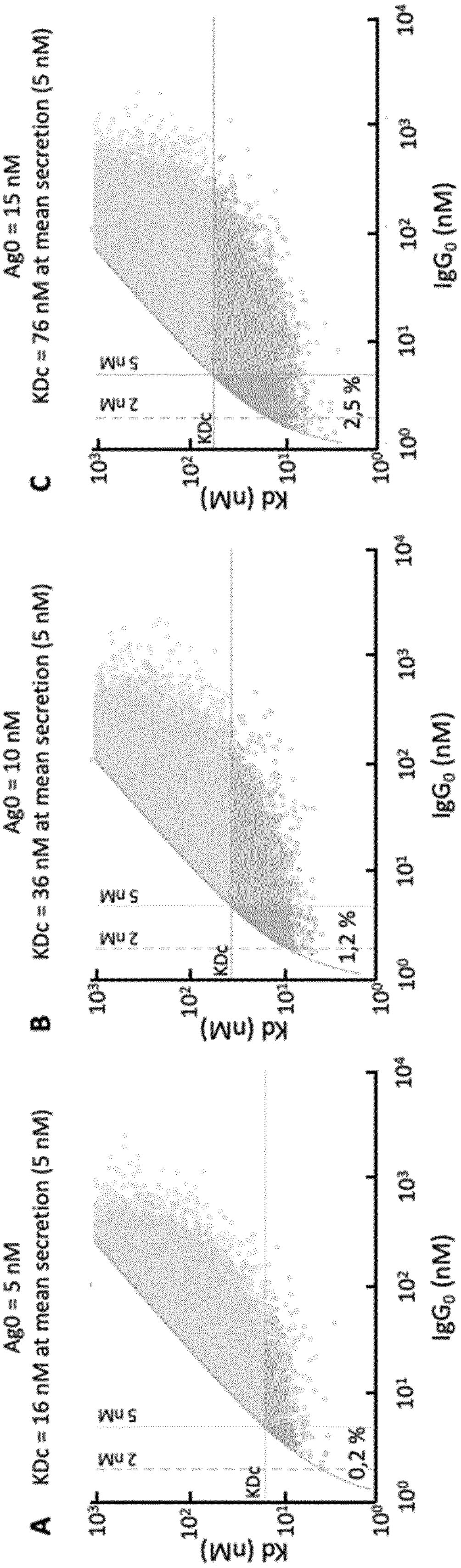
A
LOD = 1 nM
Ag0 = 5 nM
KDc = 16 nM at mean secretion (5 nM)
Kd (nM)
IgG$_0$ (nM)
2 nM
5 nM
KDc
0,2 %
B
LOD = 1 nM
Ag0 = 10 nM
KDc = 36 nM at mean secretion (5 nM)
Kd (nM)
IgG$_0$ (nM)
2 nM
5 nM
KDc
1,2 %
C
LOD = 1 nM
Ag0 = 15 nM
KDc = 76 nM at mean secretion (5 nM)
Kd (nM)
IgG$_0$ (nM)
2 nM
5 nM
KDc
2,5 %

Figure 16A-C

Europäisches Patentamt
European Patent Office
Office européen des brevets

# EUROPEAN SEARCH REPORT

Application Number
EP 24 15 4434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOUNAB YACINE ET AL: "Dynamic single-cell phenotyping of immune cells using the microfluidic platform DropMap", NATURE PROTOCOLS, NATURE PUBLISHING GROUP, GB, vol. 15, no. 9, 12 August 2020 (2020-08-12), pages 2920-2955, XP037528692, ISSN: 1754-2189, DOI: 10.1038/S41596-020-0354-0 [retrieved on 2020-08-12] | 14 | INV. G01N33/50 G01N33/557 G01N33/68 B01L3/00 |
| A | * see abstract, Figs. 1, 3, Boxes 1-3, p. 2926-2929, 2949 * | 1-13 | |
| | ----- | | |
| X | GÉRARD ANNABELLE ET AL: "High-throughput single-cell activity-based screening and sequencing of antibodies using droplet microfluidics", NATURE BIOTECHNOLOGY, NATURE PUBLISHING GROUP US, NEW YORK, vol. 38, no. 6, 30 March 2020 (2020-03-30), pages 715-721, XP037523979, ISSN: 1087-0156, DOI: 10.1038/S41587-020-0466-7 [retrieved on 2020-03-30] | 14 | TECHNICAL FIELDS SEARCHED (IPC) |
| | | | G01N B01L |
| A | * see p. 715-716, 722-724, Figs. 1-3 * | 1-13 | |
| | ----- | | |
| | -/-- | | |

1 The present search report has been drawn up for all claims

| Place of search | Date of completion of the search | Examiner |
|---|---|---|
| The Hague | 29 May 2024 | Diez Schlereth, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 4

Europäisches Patentamt
European Patent Office
Office européen des brevets

# EUROPEAN SEARCH REPORT

Application Number
EP 24 15 4434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHEMBEKAR NACHIKET ET AL: "Single-Cell Droplet Microfluidic Screening for Antibodies Specifically Binding to Target Cells", CELL REPORTS, vol. 22, no. 8, 1 February 2018 (2018-02-01), pages 2206-2215, XP093068078, US ISSN: 2211-1247, DOI: 10.1016/j.celrep.2018.01.071 Retrieved from the Internet: URL:https://pdf.sciencedirectassets.com/280959/1-s2.0-S2211124717X00090/1-s2.0-S2211124718301359/main.pdf?X-Amz-Security-Token=IQoJb3JpZ2luX2VjEMP//////////wEaCXVzLWVhc3QtMSJHMEUCIEh/sURuaNVh0xy75yp+AeDQ5eA2mPjNnnUq2YTHlQBeAiEAy4U9OBAUWQOY3o/7SPmyvZU/zMvVNcw2hQ57J8DNcBEqswUIXBAFGgwwNTkwMDM1NDY4NjUiDEm7Z> | 14 | |
| A | * see p. 2206-2207, 2211, Fig. 1 * | 1-13 | |
| X | HYUN SOO KIM ET AL: "High-throughput droplet microfluidics screening platform for selecting fast-growing and high lipid-producing microalgae from a mutant library", PLANT DIRECT, vol. 1, no. 3, 1 September 2017 (2017-09-01), pages e00011-1, XP055636150, ISSN: 2475-4455, DOI: 10.1002/pld3.11 | 14 | |
| A | * see Fig. 1, points 2.4-2.5 * | 1-13 | |

TECHNICAL FIELDS SEARCHED (IPC)

-/--

The present search report has been drawn up for all claims

1

| Place of search | Date of completion of the search | Examiner |
|---|---|---|
| The Hague | 29 May 2024 | Diez Schlereth, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 24 15 4434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | WO 2021/072306 A1 (1859 INC [US]) 15 April 2021 (2021-04-15)<br>* see Fig. 2, par. [010-015, 967-070, 073-078, 080-083, 087-088, 093, 207, 215-217, 238-239, 242, 248-258, 276] *<br>----- | 14<br><br>1-13 | |
| A | EP 3 819 637 A1 (VELABS THERAPEUTICS GMBH [DE]) 12 May 2021 (2021-05-12)<br>* see par. [001-007, 040-043, 047] *<br>----- | 1-14 | |
| A | WO 2020/127754 A1 (PARIS SCIENCES LETTRES QUARTIER LATIN [FR] ET AL.) 25 June 2020 (2020-06-25)<br>* see Results & Discussion *<br>----- | 1-14 | |
| A | IMMANUEL SANKA: "User-friendly analysis of droplet array images", ANALYTICA CHIMICA ACTA, vol. 1272, 1 September 2023 (2023-09-01), page 341397, XP093168462, AMSTERDAM, NL ISSN: 0003-2670, DOI: 10.1016/j.aca.2023.341397 Retrieved from the Internet: URL:https://pdf.sciencedirectassets.com/271374/1-s2.0-S0003267023X00336/1-s2.0-S0003267023006189/main.pdf?X-Amz-Security-Token=IQoJb3JpZ2luX2VjEI3//////////wEaCXVzLWVhc3QtMSJIMEYCIQCQlERJi+nBNFMfRIbrxDTIhnt8zzqRBZIXTHFagFuZ9QIhAJ6HLicXFuLya8KjNjEv5iTLgg38QKkrE8IeZQhPrk5BKrMFCBYQBRoMMDU5MDAzNTQ2ODY1Igw6Z><br>* see p. 3-4, 20-29, claims *<br>-/-- | 1-14 | TECHNICAL FIELDS SEARCHED (IPC) |

The present search report has been drawn up for all claims

1

| Place of search | Date of completion of the search | Examiner |
|---|---|---|
| The Hague | 29 May 2024 | Diez Schlereth, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 15 4434

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & I Sanka: "Appendix", , 30 September 2023 (2023-09-30), XP093168470, Retrieved from the Internet: URL:https://www.sciencedirect.com/science/article/pii/S0003267023006189?via%3Dihub#appsec1 ----- | | |
| A | WO 2023/281273 A1 (LIGHTCAST DISCOVERY LTD [GB]) 12 January 2023 (2023-01-12) * the whole document * ----- | 1-14 | |

TECHNICAL FIELDS SEARCHED (IPC)

The present search report has been drawn up for all claims

1

| Place of search | Date of completion of the search | Examiner |
|---|---|---|
| The Hague | 29 May 2024 | Diez Schlereth, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 15 4434

This annex lists the patent family members relating to the patent documents cited in the above-mentioned European search report. The members are as contained in the European Patent Office EDP file on The European Patent Office is in no way liable for these particulars which are merely given for the purpose of information.

29-05-2024

| Patent document cited in search report | Publication date | Patent family member(s) | Publication date |
|---|---|---|---|
| WO 2021072306 A1 | 15-04-2021 | AU 2020361681 A1 | 05-05-2022 |
| | | CA 3157359 A1 | 15-04-2021 |
| | | CN 114829626 A | 29-07-2022 |
| | | EP 4041310 A1 | 17-08-2022 |
| | | GB 2604481 A | 07-09-2022 |
| | | IL 292055 A | 01-06-2022 |
| | | JP 2022552194 A | 15-12-2022 |
| | | KR 20220097404 A | 07-07-2022 |
| | | US 2021106996 A1 | 15-04-2021 |
| | | US 2021106998 A1 | 15-04-2021 |
| | | US 2021106999 A1 | 15-04-2021 |
| | | US 2021107000 A1 | 15-04-2021 |
| | | US 2021107001 A1 | 15-04-2021 |
| | | US 2021107002 A1 | 15-04-2021 |
| | | US 2021107003 A1 | 15-04-2021 |
| | | US 2021107004 A1 | 15-04-2021 |
| | | US 2022176374 A1 | 09-06-2022 |
| | | US 2022176375 A1 | 09-06-2022 |
| | | US 2022226825 A1 | 21-07-2022 |
| | | US 2022266250 A1 | 25-08-2022 |
| | | US 2022297125 A1 | 22-09-2022 |
| | | US 2022305492 A1 | 29-09-2022 |
| | | WO 2021072306 A1 | 15-04-2021 |
| EP 3819637 A1 | 12-05-2021 | NONE | |
| WO 2020127754 A1 | 25-06-2020 | DK 3899027 T3 | 01-05-2023 |
| | | EP 3670667 A1 | 24-06-2020 |
| | | EP 3899027 A1 | 27-10-2021 |
| | | EP 4234712 A2 | 30-08-2023 |
| | | LT 3899027 T | 10-07-2023 |
| | | US 2022065849 A1 | 03-03-2022 |
| | | WO 2020127754 A1 | 25-06-2020 |
| WO 2023281273 A1 | 12-01-2023 | CN 117651613 A | 05-03-2024 |
| | | EP 4366877 A1 | 15-05-2024 |
| | | WO 2023281273 A1 | 12-01-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **RATSWOHL et al.** *Eur. J. Immunol.*, 2023, vol. 0, 2350408
- **YU et al.** *Nature*, 2023, vol. 614, 539-547
- **DUMONTET et al.** *Nat Rev Drug Discov*, 2023, vol. 22 (8), 641-661
- **GUO et al.** *RSC Adv.*, 2016, vol. 6, 13837-13845
- **LUPU et al.** *Int. J. Mol. Sci.*, 2021, vol. 22 (23), 12832
- **GÉRARD et al.** *Nat. Biotechnol.*, 2020, vol. 38 (6), 715-721
- **BUCHELI et al.** *Eur. J. Immunol.*, 2021, vol. 51 (6), 1334-1347
- **EYER et al.** *Nat. Biotechnol.*, 2017, vol. 35 (10), 977-982
- **CANALES-HERRERIAS et al.** *J. Clin. Invest.*, 2022, vol. 132 (12), e153580
- **EYER et al.** *Nature Biotechnology*, 2017, vol. 35 (10), 977-982
- **MOLARI et al.** *eLife*, 2020, vol. 9, e55678
- **NEAL et al.** *J. Immunol. Res. Ther.*, 2017, vol. 2, 68-79
- **GRUBAUGH et al.** *Vaccine*, 2013, vol. 31, 3805-3810
- **EYER et al.** *Nat. Biotech*, 2017, vol. 35 (10), 977-982
- **GÉRARD et al.** *Nature Biotechnology*, 2020, vol. 38 (6), 715-721
- **EYER et al.** *Nature*, 2017
- **EYER et al.** *Nature Biotech*, 2017
- **GÉRARD et al.** *Nature Biotech*, 2020